# EUROPEAN PATENT APPLICATION

(11) **EP 3 263 524 A1**
(43) Date of publication of application: **03.01.2018**
(21) Application number: 16754986.4
(22) Date of filing: 23.02.2016
(51) Int. Cl.: C01F 7/16, A61K 8/26, A61Q 5/06, A61Q 13/00

(54) **MAYENITE COMPOUND, MULTIFUNCTIONAL AGENT, AND PRODUCTION METHOD FOR MAYENITE-COMPOUND-CONTAINING PRODUCT**

(30) Priority: 26.02.2015 JP 2015037175; 25.01.2016 JP 2016011287
(71) Applicant: NGK Spark Plug Co., Ltd., Nagoya-shi, Aichi 467-8525 (JP)
(72) Inventor: WATANABE, Daisuke, Nagoya-shi Aichi 467-8525 (JP); ICHIMURA, Yoshiyuki, Nagoya-shi Aichi 467-8525 (JP); OGURI, Sadatoshi, Nagoya-shi Aichi 467-8525 (JP); SHINOHARA, Kota, Nagoya-shi Aichi 467-8525 (JP); SUZUKI, Tetsuro, Nagoya-shi Aichi 467-8525 (JP); HISHIKI, Makoto, Nagoya-shi Aichi 467-8525 (JP); FUJII, Yasuhiro, Nagoya-shi Aichi 467-8525 (JP)
(74) Representative: Zimmermann & Partner Patentanwälte mbB
(86) International application number: PCT/JP2016/000938
(87) International publication number: WO 2016/136235

(57) **Abstract**

Objects of the present invention are to provide a mayenite compound which can induce electron donation reaction, H⁻ donation reaction, deoxygenation reaction, and dehydration reaction; a multifunctional agent which contains the mayenite compound which is not dissolved in a solvent, and which can induce electron donation reaction, H⁻ donation reaction, deoxygenation reaction, and dehydration reaction; and a method for producing a mayenite-compound-containing product. The invention provides a mayenite compound characterized by comprising enclosing electron and H , a multifunctional agent characterized in that the agent contains at least one of the mayenite compound I and a mixture II of the mayenite compound enclosing electron and the mayenite compound enclosing H⁻, and exhibits radical scavenging activity, reducing property, deoxygenation performance, and dehydration performance; and a method for producing a mayenite-compound-containing product containing the aforementioned mayenite compound.

## Description

### TECHNICAL FIELD

The present invention relates to a mayenite compound, to a multifunctional agent containing a mayenite compound (hereinafter may be referred to as a "mayenite-compound-containing multifunctional agent), and to a method for producing a mayenite-compound-containing product. More particularly, the invention relates to a mayenite compound which can induce electron donation reaction, H⁻ donation reaction, deoxygenation reaction, and dehydration reaction; to a multifunctional agent which contains a mayenite compound, which is not dissolved in a solvent, and which exhibits radical scavenging activity, reducing property, deoxygenation performance, and dehydration performance; and to a method for producing a mayenite-compound-containing product with high purity.

### BACKGROUND ART

Hitherto, there has been known a mayenite compound, which is a compound in which an anion is enclosed in a positively ionized cage-like crystal structure. A typical example of the mayenite compound is 12CaO·7Al₂O₃, having a cage-like crystal structure of elements Ca, Al, and O. 12CaO·7Al₂O₃ may be abbreviated as C12A7.

The elements forming the cage-like crystal structure are not limited to the aforementioned three elements, and there are various mayenite compounds formed of various elements. For example, there has been well known a 12CaO·7Al₂O₃-12SrO·7Al₂O₃ mixed crystal compound, in which Ca elements forming C12A7 are partially substituted by Sr elements.

Widely known anion species enclosed in a mayenite compound are O⁻, O²⁻, OH⁻, S²⁻, F⁻, Cl⁻, H⁻, etc. There are also known mayenite compounds in which electrons are enclosed instead of anions.

Hitherto, mayenite compounds enclosing anions or electrons are known to exhibit reducing action and antioxidizing action.

For example, Patent Document 1 discloses a 12CaO·7Al₂O₃ compound containing electrons (e⁻) at an electron density of 2 × 10¹⁸ cm⁻³ or higher and lower than 2.3 × 10²¹ cm⁻³ (see claim 1, Patent Document 1). Patent Document 1 also discloses that a powder of an inorganic compound enclosing electrons exhibits excellent antioxidant function (see [0006], Patent Document 1).

Patent Document 2 discloses antioxidants each containing, as an active ingredient, a 12CaO·7Al₂O₃ compound and/or a 12SrO·7Al₂O₃ compound, and a mixed crystal thereof, having a hydrogen anion (H⁻) density of 1 × 10¹⁸ cm⁻³ or higher (see claim 1, Patent Document 2).

However, hitherto, there has been reported no mayenite compound enclosing both electron and H⁻. Also, use of a mayenite compound enclosing electron in combination with a mayenite compound enclosing H⁻ has not been reported.

By virtue of reducing action and antioxidizing action of mayenite compounds, some mayenite compounds are used as antioxidants. For example, Patent Document 2 discloses an antioxidant containing, as an active ingredient, a powder of an hydrogen-anion-containing inorganic compound (a 12CaO·7Al₂O₃ compound, a 12SrO·7Al₂O₃ compound, or a 12CaO·7Al₂O₃-12SrO·7Al₂O₃ mixed crystal compound), and use thereof (see claim 1, Patent Document 2). Patent Document 2 also discloses specific examples of the antioxidant-containing product, including an external skin agent, a cosmetic composition, a whitening agent, an antioxidant for plastics, and an antioxidant for coatings (see claims 2 to 6, Patent Document 2). Each of the antioxidant-containing products disclosed in Patent Document 2 is characterized by containing a mayenite compound as an active ingredient in a solid or a high-viscous gel. Hitherto, there has not been reported a mode of employment of a mayenite compound serving as an antioxidant by placing it in a liquid such as oil, without the mayenite compound being dissolved in the liquid.

Specific examples of the oil containing the aforementioned antioxidant include fuel oils such as petroleum, kerosene, light oil, wax, and bio fuel; organic coating materials such as ink and paint containing drying oil; food oil for cooking; cosmetic oils such as horse oil; oil for perfume; mineral oil serving as organic solvent; machine oil; lubricating oil; and insulating oil. Specific examples of the cooking food oil include vegetable oils such as sesame oil and rapeseed oil, and animal oils such as fish oil, lard, and whale oil. Specific examples of the perfume oil include essential oils (also called aroma oils) and hair oils such as a pomade. Hydrocarbons contained in the oil react with oxygen in air by the behavior of light, heat, the presence of metal, etc., to thereby form an oxidized product. When such a hydrocarbon is oxidized, coloring of the oil may occur. In addition, unpleasant odor and hazardous substances may be generated. Notably, the higher the unsaturation degree, the greater the sensibility to oxidation of hydrocarbon. Also, hydrocarbon reacts with oxygen promoted by light, heat, etc., to thereby generate peroxy radicals (also called free radicals). The thus-generated peroxy radicals are highly reactive. Through reaction with hydrocarbon, various polymers, lower fatty acids, and other species are formed. In order to prevent oxidation of hydrocarbon and radical reaction, a variety of antioxidants are added to the target oil. In addition, oil deterioration is accelerated in the presence of water. In particular cases of machine oil, lubricating oil, insulating oil, and the like, corrosion and sludge more readily occur by increase in amount of water in oil. For example, when the insulating oil is oxidized, or the oil is deteriorated by water, to thereby generate corrosion or sludge, breakdown voltage decreases, thereby possibly resulting in break down. In this way, materials such as insulating oil are deteriorated not only via oxidation of hydrocarbon but also by water in the oil. Therefore, various counter measures have been proposed for removing water from the target oil.

Examples of the antioxidant employed in the oil include a phenolic antioxidant, a phosphorus-containing antioxidant, a sulfur-containing antioxidant, and vitamin E. All these antioxidants exhibit antioxidation performance, when they are dissolved in a solvent, particularly in oil. When the antioxidant is dissolved in oil, the composition of the oil in which the antioxidant has been dissolved differs from that of the oil before incorporation of oil. The amount (or the like) of antioxidant to be dissolved in oil may be limited by certain limitations, standards, and other factors. Notably, there has not conventionally been reported an antioxidant which does not dissolve in a solvent (e.g., oil) and which can prevent oxidation of substances in a target solution.

To food oil, an antioxidant is generally added to prevent oxidation of hydrocarbon. However, according to the relevant regulation, the type, amount, and the like of the antioxidant to be added to the food oil are regulated so as not to alter the ingredients of the food. More specifically, under regulation the JAS standards, one example of the antioxidant which can be added to food is vitamin E originating from olive oil. However, the amount of vitamin E added to food oil in an amount according to the conventional regulation is unsatisfactory, and difficulty is encountered in completely preventing oxidation of hydrocarbon, which is problematic. Also, vitamin E contained in the food decomposes via oxidation as time elapses, and the vitamin E content gradually lowers. As a result, nutrients contained in food oil problematically decrease.

In order to prevent deterioration of oil due to oxidation of hydrocarbon, an antioxidant is generally added to fuel oil (e.g., petroleum) during long-term storage. Conventionally, a phenol-based antioxidant is generally used in fuel oil. However, since such an antioxidant as employed in fuel oil is dissolved in the oil and modifies the composition of the oil, the allowable amount of a phenol-based antioxidant added to fuel oil is regulated. In accordance with the regulated antioxidant level, the phenol-based antioxidant fails to be added to fuel oil in such an amount as to completely prevent oxidation. As a result, in some cases, oxidation and deterioration of fuel oil cannot sufficiently be prevented.

Another antioxidant to be added to fuel oil is a sulfur-containing antioxidant. The sulfur-containing antioxidant generates sulfur oxide (SOₓ)--a substance causing acid rain--during combustion reaction of fuel oil. In order to reduce the SOₓ level of exhaust gas during combustion, addition of the sulfur-containing antioxidant is also regulated. Thus, similarly, the sulfur-containing antioxidant fails to be added to fuel oil in such an amount as to completely prevent oxidation, whereby oxidation and deterioration of fuel oil cannot sufficiently be prevented, which is problematic.

Organic coatings such as ink and paint may be deteriorated during storage thereof in a storage container, due to oxidation of a hydrocarbon serving as a coating ingredient. Through such deterioration, problematically, the color tone of the coating varies, and coating failures such as cracking and peeling tend to occur after application thereof. Conventionally, for preventing deterioration of an organic coating due to oxidation during storage thereof, various antioxidants are added to and dissolved in organic coatings. However, such an antioxidant as added to an organic coating modifies the composition of the coating ingredients, thereby possibly changing the subtle color tone of ink, paint, etc. Thus, difficulty is encountered in adding and dissolving an antioxidant to and in an organic coating (e.g., ink or paint) in such an amount as to completely prevent oxidation of hydrocarbon, which is problematic.

Hitherto, there has never been known use of a mayenite compound as a reducing agent for modifying waste oil in which hydrocarbons are in an oxidized state. Also, use of a mayenite compound for preventing oxidation of oil or removing water from oil has never been reported.

Patent Document 3 discloses that electrons in an electride compound are substituted by anions through thermal treatment in an oxygen, hydrogen, or nitrogen atmosphere, to thereby enclose O⁻, H⁻, or N⁻ in the compound (see p. 6, line 22 to p. 7 line 2, and P. 15, lines 3 to 11, Patent Document 3). Patent Document 3 also discloses that C12A7 compounds and homologous compounds each enclosing O⁻ ions can be used as an oxidation catalyst and an O⁻ beam-generating material (see P. 15, lines 12 and 13, Patent Document 3).

Furthermore, hitherto, there has not been reported use of a mayenite compound as an oxidizing agent or a reducing agent in a gas phase containing a hazardous substance, a malodorous substance, etc., so as to modify the gas phase.

In addition, there has not been reported that a mayenite compound which encloses active oxygen species such as O⁻ and O₂⁻ has an antibacterial action to bacteria and viruses and can be used as an antibacterial agent.

### PRIOR ART DOUMENTS

### PATENT DOCUMENTS

Patent Document 1 : JP-A-2009-161728
Patent Document 2 : WO2008/087774 A1
Patent Document 3 : WO2005/000741 A1

### SUMMARY OF THE INVENTION

### PROBLEM TO BE SOLVED BY THE INVENTION

Meanwhile, mayenite compounds have various functions depending on the types of species (i.e., electron and anions) enclosed in each mayenite compound. Thus, such mayenite compound can conceivably find a variety of uses in accordance with the diverse functions of mayenite compounds enclosing different types of anions and the like. Also, when mayenite compounds having different functions are used in combination, such combinations may find a wide variety of uses. Furthermore, by virtue of synergistic functions, such a combination is conceivably well-suited for a certain use.

An object of the present invention is to provide a mayenite compound which can induce electron donation reaction, H⁻ donation reaction, deoxygenation reaction, and dehydration reaction.

Another object of the present invention is to provide a multifunctional agent which contains a mayenite compound, which is not dissolved in a solvent such as oil, and which can induce electron donation reaction, H⁻ donation reaction, deoxygenation reaction, and dehydration reaction; i.e., a multifunctional agent exhibiting radical scavenging activity, reducing property, deoxygenation performance, and dehydration performance.

Still another object of the present invention is to provide a container, a porous body, a coating film, a filter, and a reactor, each exhibiting radical scavenging activity, reducing property, deoxygenation performance, and dehydration performance.

Still another object of the present invention is to provide a multifunctional agent for oil which agent can maintain quality of oil, particularly insulating oil.

Still another object of the present invention is to provide a method for using a multifunctional agent which can maintain quality of insulating oil.

Yet another object of the present invention is to provide an oil-immersed transformer containing an insulating oil whose quality is maintained.

Still another object of the present invention is to provide an oil-impregnated capacitor containing an insulating oil whose quality is maintained.

Still another object of the present invention is to provide a gas phase-modifying agent which can modify a gas phase.

Yet another object of the present invention is to provide a tobacco smoke filter which can modify tobacco smoke.

Still another object of the present invention is to provide an attachment for tobacco smoke which can modify tobacco smoke.

Still another object of the present invention is to provide a mask having radical scavenging activity, reducing property, and dehydration performance.

Yet another object of the present invention is to provide a method for yielding a mayenite-compound-containing product, which method can yield a mayenite-compound-containing product which contains a high-purity mayenite compound, with deterioration of the product being prevented.

Still another object of the present invention is to provide a method for yielding a mayenite-compound-containing product, which method can produce a mayenite compound I enclosing electron and H⁻ at high densities, a mayenite compound enclosing electron at high density, or a mayenite compound enclosing H⁻ at high density, to thereby yield a mayenite-compound-containing product containing at least one of the mayenite compound I enclosing electron and H⁻ at high densities and a mixture II of the mayenite compound enclosing electron at high density and the mayenite compound enclosing H⁻ at high density.

### MEANS FOR SOLVING THE PROBLMEM

Means for solving the aforementioned problems are as follows.
(1) A mayenite compound characterized by enclosing electron and H⁻.
(2) A multifunctional agent, characterized by containing at least one of a mayenite compound I enclosing electron and H⁻, and a mixture II of a mayenite compound enclosing electron and a mayenite compound enclosing H , and exhibiting radical scavenging activity, reducing property, deoxygenation performance, and dehydration performance.
(3) A container, characterized by containing, at least as a part thereof, a multifunctional agent as recited in (2) above.
(4) A container according to (3) above, which is made of a ceramic or a glass.
(5) A porous body, characterized by comprising a multifunctional agent as recited in (2) above, and having at least a porous surface.
(6) A coating film, characterized by comprising a multi-functional agent as recited in (2) above.
(7) A filter, characterized by comprising a multifunctional agent as recited in (2) above.
(8) A reactor, characterized by comprising a casing at least having two openings and an inner space, and a multifunctional agent as recited in (2) above disposed in the inner space.
(9) A multifunctional agent for oil, characterized by comprising a multifunctional agent as recited in (2) above for use in maintaining the quality of the oil.
(10) A multifunctional agent for oil according to (9) above, wherein the oil is an insulating oil.
(11) A method of using a multifunctional agent, characterized in that the method comprises using a multifunctional agent as recited in (2) above which is placed at least in the insulating oil or a site in the vicinity of the insulating oil.
(12) An oil-immersed transformer comprising a transformer tank and an insulating oil charged in the transformer tank, characterized in that the multifunctional agent as recited in (2) above is located at least in the insulating oil or a site in the vicinity of the insulating oil.
(13) An oil-impregnated capacitor comprising a capacitor tank for accommodating the capacitor, and an insulating oil charged in the capacitor tank, characterized in that the multifunctional agent as recited in (2) above is located at least in the insulating oil or a site in the vicinity of the insulating oil.
(14) A gas phase-modifying agent, characterized by comprising a multifunctional agent as recited in (2) above being used for modifying a gas phase.
(15) A tobacco smoke filter, characterized by comprising a multifunctional agent as recited in (2) above, to thereby modify tobacco smoke.
(16) An attachment for tobacco smoke, characterized by having a casing at least having two openings and an inner space, and a multifunctional agent as recited in (2) above disposed in the inner space, wherein a tobacco piece is attached to one of the openings, and the other opening serves as a mouth piece, and tobacco smoke which has been passed through the casing is to be modified.
(17) A mask, characterized by having a filter as recited in (7) above and a reactor as recited in (8) above.
(18) A method for yielding a mayenite-compound-containing product containing a multifunctional agent as recited in claim 2, wherein the method comprises at least a mixing and pulverization step and a second firing step among a mixing and pulverization step of mixing and crushing raw materials by means a mixing and pulverization tool, to thereby obtain a mixture; a firing step of firing the mixture placed in or on a firing tool, to thereby obtain a mayenite compound precursor; a molding step of molding the mixture or the mayenite compound precursor placed in a mold, to thereby form a compact; and a second firing step of firing the mixture, compact, or a mayenite compound precursor placed in or on a firing tool, in a reducing atmosphere or in a inert gas; wherein at least one of the mixing and pulverization tool, molding tool, and firing tool is made of the mayenite compound.
(19) A method for producing a mayenite-compound-containing product according to (18) above, characterized in that, in the second firing step, at least one of a dehydrating agent and a deoxygenating agent is placed in at least one of a furnace and a gas feed path to the furnace, to thereby conduct firing in at least one of a dry atmosphere and a deoxygenated atmosphere.

### EFFECTS OF THE INVENTION

Means (1) above can provide a mayenite compound having such a particular crystal structure enclosing electron and H⁻, thereby inducing electron donation reaction, H⁻ donation reaction, deoxygenation reaction, and dehydration reaction.

The mayenite compound I and the mixture II, each enclosing electron, have electron donating property, to thereby scavenge free radicals, in particular active oxygen, and reducing property. The mayenite compound I and the mixture II, each enclosing H⁻, have H⁻ donating property and reducing property. The mayenite compound I and the mixture II according to the present invention have a cage-like crystal structure. The positively charged inclusion cage encloses at least one negatively charged species such as electron and H⁻. The inclusion cage has such a function that it undergoes hydration reaction via contact with water, to thereby remove water present around the cage and incorporate anions, in particular oxygen ions, thereinto. Differing from conventionally used electron donating agents formed of an organic compound, the mayenite compound according to the present invention is a ceramic material formed of an inorganic compound. The mayenite compound, which is a ceramic material, is not dissolved in an organic solvent, particularly in oil. Means (2) above can provide a multifunctional agent which is not dissolved in a liquid such as oil. The multifunctional agent exhibits radical scavenging activity and reducing property (by donating electrons to an ingredient in the liquid); reducing property (by donating to H⁻ an ingredient in the liquid); deoxygenation performance (by incorporating anions, particularly oxygen ions, into the inclusion cage); and dehydration performance (through hydrogenation reaction via contact with water).

Means (3) to (8) can provide a container, a porous body, a coating film, a filter, and a reactor, each exhibiting radical scavenging activity, reducing property, deoxygenation performance, and dehydration performance.

Means (9) and (10) can provide a multifunctional agent for oil which agent can maintain the quality of the oil, particularly an insulating oil.

Means (11) can provide a method of using a multifunctional agent which can maintain the quality of insulating oil.

Means (12) can provide an oil-immersed transformer, in which the quality of insulating oil is maintained.

Means (13) can provide an oil-impregnated capacitor, in which the quality of insulating oil is maintained.

Means (14) can provide a gas phase-modifying agent which can modify a gas phase.

Means (15) can provide a tobacco smoke filter which can modify tobacco smoke.

Means (16) can provide an attachment for tobacco smoke which can modify tobacco smoke.

Means (17) can provide a mask which has radical scavenging activity, reducing property, and dehydration performance.

Means (18) can provide a method for producing a mayenite-compound-containing product which contains a high-purity mayenite compound, with deterioration of the product being prevented.

Means (19) can provide a method for yielding a mayenite-compound-containing product, which method can produce a mayenite compound I enclosing electron and H⁻ at high densities, a mayenite compound enclosing electron at high density, or a mayenite compound enclosing H⁻ at high density, to thereby yield a mayenite-compound-containing product containing at least one of the mayenite compound I enclosing electron and H⁻ at high densities and a mixture II of the mayenite compound enclosing electron at high density and the mayenite compound enclosing H⁻ at high density.

### BRIEF DESCRIPTION OF THE DRAWINGS

[FIG. 1] A schematic view of the crystal structure of a mayenite compound.
[FIG. 2] A sketch of a multifunctional agent in granular form according to the present invention.
[FIG. 3] A sketch of a multifunctional agent in ball form according to the present invention.
[FIG. 4] A sketch of a multifunctional agent in filter form according to the present invention.
[FIG. 5] A sketch of a multifunctional agent in coating film form according to the present invention.
[FIG. 6] A sketch of a multifunctional agent in container form according to the present invention.
[FIG. 7] A sketch of a multifunctional agent in a form of attachment for tobacco smoke according to the present invention.
[FIG. 8] A sketch of a multifunctional agent in reactor form according to the present invention.
[FIG. 9] A sketch of an example of an oil-immersed transformer.
[FIG. 10] A sketch of an example of an oil-impregnated capacitor.
[FIG. 11] An X-ray diffraction pattern of sample B of an Example.

### MODES FOR CARRYING OUT THE INVENTION

### Mayenite compound enclosing both electron and H⁻

Firstly, the mayenite compound according to the present invention enclosing both electron and H⁻ (hereinafter may be referred to as simply "mayenite compound I") will be described in detail.

The mayenite compound is formed of a positively charged inclusion cage, and a negatively charged particle is enclosed in the cage. In the mayenite compound I, the enclosed particles are electron and H⁻. Since the inclusion cages form a 3-dimensionally linked steric network, the entire cage cluster structure is realized.

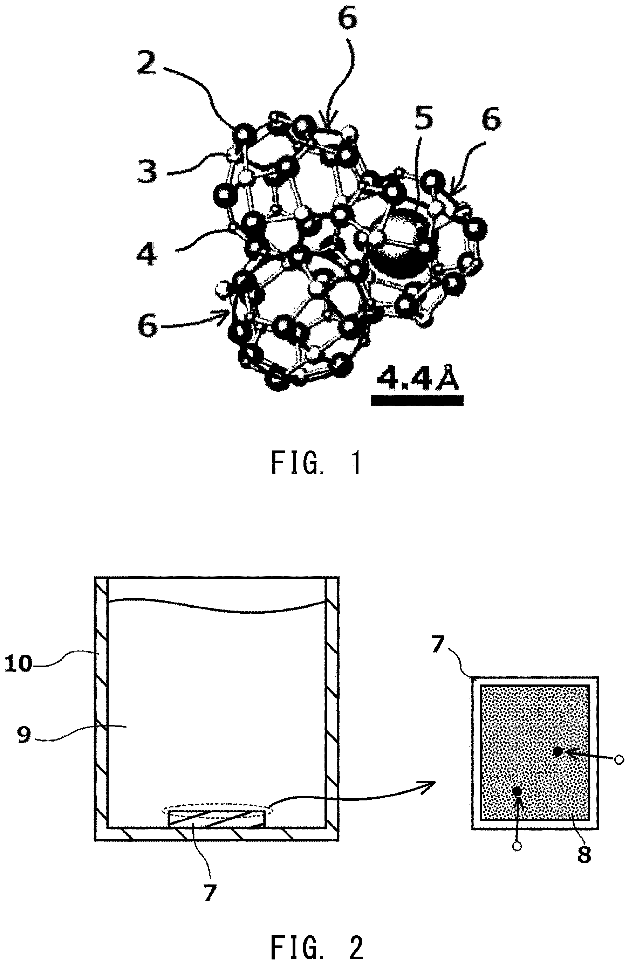

The inclusion cage and a cage cluster formed of a plurality of inclusion cages have a crystal structure characteristic to a mayenite compound. As shown in FIG. 1, the unit inclusion cage is mainly formed of three kinds of elements having different sizes. The inclusion cage shown in FIG. 1 is formed of elements. In FIG. 1, reference numeral 4 denotes Al, 3 denotes Ca, and 2 denotes O. The mayenite compound having an inclusion cage formed of Al, Ca, and O may be represented by 12CaO·7Al₂O₃ or C12A7. The cage cluster shown in FIG. 1 is formed of three inclusion cages 6. C12A7 has 12 inclusion cages in a unit lattice. For the purpose of convenience, an inclusion cage on the right side encloses one electron. However, in an actual state, electrons serving as enclosed particles have a very small mass as compared with ions, and electrons behave quantum-mechanically. That is, although electrons are withdrawn to positive charges, electrons are delocalized and thus are identified merely as existence probability.

So long as the crystal structure characteristic to the mayenite compound is not broken, the inclusion cage may contain an element other than Al, Ca, and O. For example, Al atoms may be entirely or partially substituted by atoms of at least one element selected from the group consisting of B, Ga, C, Si, Fe, and Ge. Ca atoms may be entirely or partially substituted by atoms of at least one element selected from the group consisting of Sr, Li, Na, K, Mg, Sr, Ba, Sc, Ti, V, Cr, Mn, Fe, Co, Ni, Cu, Ir, Ru, Rh, and Pt. O atoms may be entirely or partially substituted by atoms of at least one element selected from the group consisting of H, F, Cl, Br, and Au. As is known in the art, through substitution of Al, Ca, or O by other elements, interatomic distances are changed, to thereby modify the properties of a mayenite compound. When substitution of the above elements occurs in the mayenite compound, the effects of the present invention can be attained, without substantially increasing the size of the crystal structure.

One enclosed particle is encapsulated by one inclusion cage forming the cage cluster. C12A7 has 12 inclusion cages in a unit lattice and positively charged with a total valence of 4. Thus, when the particle enclosed by an inclusion cage is a divalent anion, anions are enclosed by two of the 12 inclusion cages, whereas when the particle enclosed by an inclusion cage is a monovalent anion, anions are enclosed by four of the 12 inclusion cages. Also, when the particle enclosed by an inclusion cage is an electron, as described above, the electron is delocalized and thus is identified merely as existence probability. Negatively charged particles can consistently be retained in positively charged inclusion cages.

The mayenite compound I encloses electron and H⁻, and an electron and H⁻ each have a monovalent negative charge. Thus, in one case, three of the 12 inclusion cages enclose H⁻, and one electron is present in a delocalized manner. In another case, H⁻ is enclosed by two of the 12 inclusion cages, and two electrons are present in a delocalized manner. In still another case, H⁻ is enclosed by one of the 12 inclusion cages, and three electrons are present in a delocalized manner. In the mayenite compound I, no particular limitation is imposed on the type of enclosed particles in individual inclusion cages, so long as electron and H⁻ are enclosed in any of the cage cluster forming the mayenite compound I.

No particular limitation is imposed on the ratio in amount of electron to H⁻, two components being enclosed in the mayenite compound I. The ratio in amount of electron to H⁻ may be appropriately modified such that reduction reaction induced by the mayenite compound I can be appropriately controlled, and that the mayenite compound I can have sufficient electron donation capacity and H⁻ donation capacity for serving as an antioxidant, a reducing agent, or the like. The mayenite compound I is formed of one or more cage clusters.

In addition to electron and H⁻, the mayenite compound I may further contain an anion other than H⁻, a radical species, etc. In other words, the mayenite compound according to the present invention may contain anions and radicals enclosed in 2 or fewer inclusion cages, so long as at least one electron and at least one H⁻ are enclosed in 12 inclusion cages. Specific examples of the anion other than H⁻ which may be contained in the mayenite compound I include S²⁻, F⁻, Cl⁻, O⁻, O²⁻, O₂²⁻, and OH⁻.

Whether the mayenite compound I encloses electrons can be confirmed by the presence of two different optical absorption peaks at photon energies of 0.4 eV and 2.8 eV in an optical absorption spectrum. The amount of enclosed electrons may be determined from the peak intensities. Alternatively, whether the mayenite compound I encloses electrons may be confirmed by the presence of a signal attributed to electron in an ESR spectrum measured by means of an electron spin resonance (ESR) spectrometer. Also, whether the mayenite compound I encloses H⁻ may be confirmed through secondary ion mass spectrometry (SIMS). The H⁻ concentration may be also determined through SIMS. Alternatively, whether the mayenite compound I encloses H⁻ may be confirmed by checking generation of hydrogen gas when the mayenite compound I is dissolved in a strong acid such as hydrochloric acid, or when the crystal structure of the mayenite compound I is broken through hydration by immersing the compound in water. Still alternatively, whether the mayenite compound I encloses H⁻ may be confirmed by means of a nuclear magnetic resonance (NMR) spectrometer.

The mayenite compound I, which encloses both electrons and H , exhibits sufficient electron donating property and H⁻ donating property. In addition, by virtue of the particular crystal structure as described above, the mayenite compound I actively incorporates anions, in particular oxygen ions, and undergoes hydration reaction via contact with water. Therefore, the mayenite compound I can induce electron donation reaction, H⁻ donation reaction, deoxygenation reaction, and dehydration reaction.

The mayenite compound I has both a function of a mayenite compound enclosing only electron and a function of a mayenite compound enclosing only H⁻. A mayenite compound enclosing only electron has electron donating property, to thereby scavenge free radicals, in particular active oxygen, and reducing property. A mayenite compound enclosing only H⁻ has H⁻ donating property and reducing property. The mayenite compound I has a cage-like crystal structure, where at least one of electron and H⁻ (negatively charged species) is enclosed in positively charged inclusion cages. The mayenite compound I, having a crystal lattice including at least Ca, Al, and O, can incorporate anions, in particular oxygen ions, into the inclusion cages and can remove water present around the cages via hydration reaction via contact with water. When oxygen or water is present around the mayenite compound I, firstly, the compound I incorporates oxygen ions O²⁻ into inclusion cages. Then, the incorporated oxygen ions O²⁻ react with water around the compound I, to thereby form OH⁻ in the cages. In this way, the mayenite compound I is involved in deoxygenation reaction and dehydration reaction. Differing from conventionally used electron donating agents formed of an organic compound, the mayenite compound I is a ceramic material formed of an inorganic compound. The mayenite compound, which is a ceramic material, is not dissolved in an organic solvent, particularly in oil. Thus, the mayenite compound I is not dissolved in a liquid such as oil and exhibits multiple functions: radical scavenging activity and reducing property (by donating electrons to an ingredient in the liquid); reducing property (by donating H⁻ to an ingredient in the liquid); dehydration performance (through hydrogenation reaction via contact with water); and deoxygenation performance (by incorporating anions, particularly oxygen ions, into the inclusion cage).

### Mixture II of mayenite compound enclosing electron and mayenite compound enclosing H⁻

Next, the mixture II of a mayenite compound enclosing electron and a mayenite compound enclosing H⁻ (hereinafter may be referred to as simply "mixture II") will next be described in detail. The mayenite compound which the electron is enclosed in an inclusion cage has the same structure as that of the mayenite compound I enclosing electron and H⁻, except that electron is a particle-form enclosed species of the mayenite compound. Except that the mayenite compound enclosing H⁻ as a particle-form enclosed species is H⁻, the compound has the same structure as that of the mayenite compound I enclosing electron and H⁻. In the mixture II, no particular limitation is imposed on the ratio in amount of the mayenite compound enclosing electron and that enclosing H⁻. In accordance with use of the mixture II, the ratio in amount of the mayenite compound enclosing electron and that enclosing H⁻ may be appropriately modified. Similar to the mayenite compound I, the mayenite compound enclosing electron and H⁻ may include, as unavoidable impurities, radicals, anions other than electrons and H⁻, etc.

The mixture II has the same functions as those of the mayenite compound I.

### Mayenite compound enclosing electron

The mayenite compound enclosing electron will next be described in detail. The mayenite compound enclosing electron has the same structure as that of a mayenite compound I enclosing electron and H⁻, except that the mayenite compound enclosing electron as a particle-form enclosed species. Similar to the mayenite compound I, the mayenite compound enclosing electron may further contain an anion, a radical species, etc., as unavoidable impurities.

The mayenite compound enclosing electron has radical scavenging activity (i.e., scavenging free radicals, in particular active oxygen, by virtue of electron donating property) and reducing property.

The mayenite compound enclosing electron exhibits electric conductivity via transfer of electrons between inclusion cages. As a result, the mayenite compound I and the mayenite compound enclosing electron may be used as a material of forming a member which must have electric conductivity. For example, the mayenite compound I and the mayenite compound enclosing electron may be employed as materials of electrodes for use in capacitors, batteries, etc., materials for producing electronic circuits, etc. More specifically, when a conductive material containing the mayenite compound I and the mayenite compound enclosing electron is printed onto a patterned substrate, an electronic circuit allowing electric conduction along the pattern disposed on the substrate can be yielded. Also, the mayenite compound I or the mayenite compound enclosing electron may be used as a material of a negative for emitting electrons in an electron gun.

The mayenite compound I and the mayenite compound enclosing electron exhibit electron donating property. In one possible example of electron donation reaction, the enclosed electrons are directly transferred to particles such as ions, radicals, etc. present outside the cage clusters.

### Mayenite compound enclosing H⁻

Next, the mayenite compound enclosing H⁻ will be described in detail. The mayenite compound enclosing H⁻ has the same structure as that of the mayenite compound I enclosing electron and H⁻, except that the particle species enclosed in an inclusion cage is H⁻. Similar to the mayenite compound I, the mayenite compound enclosing H⁻ may include, as unavoidable impurities, radicals, anions other than H⁻, etc.

The mayenite compound enclosing H⁻ has H⁻ donating property and reducing property.

### Forms of mayenite compound I and mixture II

The mayenite compound I or the mixture II may be in any form. Examples of the form include container, porous body, coating film, powder, granule, ball, pellet, and filter.

No particular limitation is imposed on the shape of the container. The entirety of the container may be formed of the mayenite compound I or the mixture II, or a part of the container may be formed of the mayenite compound I or the mixture II. In specific embodiments, only the inner surface(s) of the container, the bottom of the container, or the back surface of the cap of the container is formed of the mayenite compound I or the mixture II. The container having at least a part formed of the mayenite compound I or the mixture II exhibits radical scavenging activity, reducing property, deoxygenation performance, and dehydration performance. Thus, such a container can be suitably employed for long-term storage of a substance whose oxidation or water absorption is to be prevented. The container may be formed of a ceramic material or a glass material. The container made of a ceramic material becomes porous through biscuit firing. The thus-fired container has large specific surface area, leading to enhanced physical adsorption performance. In this case, through coating the surface of the container with a glaze or the like, leakage of the contents can be prevented. As the enclosed electron concentration increases, the mayenite compound enclosing electron is colored to green-to-black. Thus, by appropriately tuning the electron concentration of the mayenite compound I or the electron-enclosing mayenite compound concentration of the mixture II, the glass-made container may be used as a shading bottle.

The porous body formed of the mayenite compound I or the mixture II has an increased specific surface area, thereby providing radical scavenging activity, reducing property, deoxygenation performance, and dehydration performance, as well as enhanced physical adsorption performance. When the physical adsorption performance of the mayenite compound I or the mixture II is enhanced, the compound or mixture can remove odorous substances and impurities and possess enhanced reactivity. No particular limitation is imposed on the shape of the porous body, and any shape may be adapted. The porous body may be formed of the mayenite compound I or the mixture II, as a single material, and, optionally, an additional material such as a ceramic material. The porous body includes a porous portion at least as a surface thereof. The method for producing the porous body will be described hereinbelow. When the mayenite compound I or the mixture II is mixed with a substance having a large specific surface area (e.g., activated carbon, active alumina, or zeolite), and the resultant mixture is molded into a porous body, the porous body or a sintered product thereof exhibits higher physical adsorption performance.

The above-mentioned coating film, when formed in a target object, imparts radical scavenging activity, reducing property, deoxygenation performance, and dehydration performance to the target object. The coating film may be formed on at least a part of the target object, or on the entire surface of the object. The coating film may be formed of the mayenite compound I or the mixture II, as a single material, and, optionally, an additional material such as a ceramic material. The method for producing the coating film will be described hereinbelow.

The powder-form, granule-form, ball-form, or pellet-form mayenite compound I or mixture II has a specific surface area larger than that of the compact-form mayenite compound I or mixture II. Thus, the radical scavenging activity, reducing property, deoxygenation performance, and dehydration performance can be more effectively attained. When the mayenite compound I or the mixture II is dispersed in a target substance, or placed in the target substance in a bag or the like, oxidation and intrusion of water can be prevented, to thereby ensure the quality of the target substance. Also, the mayenite compound I or the mixture II may be directly sprayed onto an insoluble liquid such as oil.

The aforementioned filter has a shape of sheet, plate, column, or the like. The filter is placed in, for example, a tube-like casing, and a reaction substance is caused to pass through the casing from one end of the filter to the other end thereof, whereby a reaction product in the form of liquid or gas can be yielded through radical scavenging, reduction, deoxygenation, dehydration, and the like. The filter may be provided such that liquid or gas is allowed to pass through the filter. The filter may be formed only of the mayenite compound I or the mixture II, to thereby form a porous body. Alternatively, the mayenite compound I or the mixture II in powder form may be deposited on a cloth, a nonwoven fabric support obtained from natural fiber, chemical fiber, etc., or a similar support.

### Production methods for mayenite compounds I and II

Next, a method for producing the mayenite compound I and that for the mixture II will be described.

### Method for producing oxygen-containing anions

A mayenite compound precursor enclosing O-containing anion (such as O₂⁻ or OH⁻) (hereinafter may be referred to simply as a "mayenite compound precursor" is produced. Firstly, a mixing and pulverization step is conducted. In this step, raw material powders are provided at compositional proportions so as to yield a target mayenite compound, and the powders are mixed and crushed,the powder mixture is optionally spray-dried. Subsequently, a first firing step is conducted. [Mixing and pulverization step] In order to produce 12CaO·7Al₂O₃, a powder of a Ca containing compound (e.g., calcium carbonate) and a powder of Al compound (e.g., aluminum oxide) may be used as raw materials. The Ca containing compound and the Al containing compound are mixed so as to attain an element ratio Ca : Al of 12 : 14.

In one mode of the mixing and pulverization step, raw materials and balls are fed to a kneader, and the raw materials are kneaded and pulverized. The mixing/pulverization tools employed in mixing and pulverization (e.g., a mixer and balls) are preferably formed of a ceramic material having the same composition as (or a composition similar to) that of a production target mayenite compound. In the step of mixing and crushing raw material powders, the mixer and the balls wear to release some ingredients of the material thereof. Such ingredients may be incorporated into the raw materials, to thereby possibly reduce the purity of the raw materials. In contrast, in the case where the mixing/pulverization tools are formed of a ceramic material having the same composition as (or a composition similar to) that of a production target mayenite compound, a drop in purity of the raw materials can be prevented. That is, even when the above ingredients forming the mixer and balls are incorporated into the raw materials, the ingredients are the same (or similar to) those of the production target mayenite compound.

The mixing and pulverization step may be conducted by adding a solvent such as alcohol to the raw material powders, to thereby form a slurry. In this case, the slurry is rapidly dried by splaying it into a gas phase by means of a spray dryer, to thereby form a powder mixture. The gas may be a dry gas. Examples of the dry gas include air, nitrogen, and a reducing gas (e.g., hydrogen or carbon monoxide).

[First firing step] The thus-obtained powder mixture is fired in, or placed on and fired in, a firing tool such as a crucible, a firing plate, a firing sheathe, or a firing furnace, at 1,200°C or higher and lower than 1,450°C under atmospheric conditions, to thereby produce a mayenite compound precursor. The firing tools are preferably formed of a ceramic material having the same composition as (or a composition similar to) that of a production target mayenite compound. In the step of firing the powder mixture, some ingredients of the materials forming the firing tools may be incorporated into the powder mixture. Also, such ingredients may react with a material forming the firing tool at a site being in contact with the powder mixture, to thereby possibly yield a compound differing from the target mayenite compound. In contrast, in the case where the firing tools are formed of a ceramic material having the same composition as (or a composition similar to) that of a production target mayenite compound, a drop in purity of the powder mixture can be prevented. That is, even when the above ingredients forming the firing tools are incorporated into the raw materials, or reacted with the raw material powder, formation or a compound differing from the target mayenite compound is prevented.

Alternatively, the mayenite compound precursor may be produced from a sintered body obtained by sintering a powder mixture at 1,200°C or higher and lower than 1,450°C, through zone melting. Specifically, while an IR beam is focused onto the aforementioned sintered body in rod form, the sintered body is gradually pulled, whereby the IR zone melting region moves. During the movement, a single crystal of the mayenite precursor grows at an interface between the IR beam melting zone and the solidification part therearound. As a result, there can be yielded a precursor in which a mayenite compound enclosing oxygen ion O₂⁻ is provided in an inclusion cage. The mayenite compound precursor formed produced through zone melting has a rod shape. In accordance with the shape of interest of the mayenite compound, the rod-shape mayenite compound precursor may be subjected to processing (e.g., cutting or pulverizing). In one specific mode, for producing a granular-form mayenite compound I, the mayenite compound precursor may be crushed to powder by means of a ball mill or the like.

Still alternatively, the mayenite compound precursor may be produced through a chemical solution technique (sol-gel method). In one procedure, aluminum sec-butoxide, metallic Ca, and 2-methoxyethanol are mixed together, and the mixture is refluxed at 125°C for about 12 hours, to thereby yield a precursor solution. The precursor solution is applied on to a substrate through a coating technique (e.g., spin coating), and the coating is dried at 150°C for a specific period of time. Subsequently, the coating is calcined at 350°C for a specific period of time and subjected to rapid annealing at 1,000°C repeatedly, to thereby form a thin-film mayenite compound precursor. Alternatively, through placing the precursor solution in a container having a specific shape, and subjecting a thermal treatment at an appropriate temperature for an appropriate time, a bulk mayenite compound precursor can be formed.

For incorporating an element other than Ca, Al, and O, into the mayenite compound, a source powder other than calcium carbonate powder or aluminum oxide powder is added to the raw material powder. In one specific mode, for yielding a mixed crystal compound of 12CaO·7Al₂O₃ and 12SrO·7Al₂O₃, a raw material powder containing strontium is added to calcium carbonate powder or aluminum oxide powder, and the resultant powder mixture is sintered.

### Production of mayenite compound I enclosing electron and H⁻ and mixture II

Subsequently, the mayenite compound precursor in powder or granular form, as is or after a molding step, is subjected to the below-mentioned second firing step, to thereby substitute enclosed O-containing anions (e.g., O₂⁻ and OH⁻) by other ions such as H⁻ or electrons.

[Molding step] In production of a shaped product of the mayenite compound I or the mixture II, firstly, a mayenite compound precursor in powder or granular form; a mixture containing a mayenite compound precursor in powder or granular form and an optional ceramic powder; a mayenite powder such as an electron- and/or H⁻-enclosing mayenite compound powder, which has been obtained by firing a mayenite compound precursor in the manner mentioned below; or a powder mixture obtained in the mixing and pulverization step is molded, to thereby form a compact (i.e., molding step), and then the compact is fired.

No particular limitation is imposed on the method of forming a compact, and any known molding method may be employed. Examples of the molding method include press molding, isostatic pressing, rotation molding, extrusion molding, injection molding, cast molding, press-cast molding, rotation-cast molding, potter's wheel molding, thin sheet molding, and 3-dimensional laminating shaping.

In one mode of press molding, a granular mayenite powder is charged into a metal mold or a rubber mold and subjected to press molding. Press molding is suitably employed for mass-producing compacts having the same shape.

In in one mode of isostatic pressing, a mayenite powder is charged into a rubber mold and molded through application of hydrostatic pressure.

In one mode of rotation molding, a mayenite powder is fed into a rotating machine having a shape such as a drum, and the powder is granulated (i.e., undergoes particle growth) through rotation, to thereby yield a structure having a shape (spherical, pellet-like, or the like).

In one mode of extrusion molding, a solvent, a binder, or the like is added to a mayenite powder, and a mixture having plasticity is extruded via a metal mold through pressing by means of an extruder, to thereby yield a compact having a shape (rod, cylinder, or the like). Through cutting a compact extruded through a metal mold, a pellet-form compact may be formed.

In one mode of injection molding, a resin or a similar material is added to a mayenite powder, and a mixture having plasticity is injected into a metal mold. Injection molding is suitably employed for producing compacts having a complex shape.

In one mode of cast molding, press-cast molding, or rotation-cast molding, a solvent (e.g., alcohol) is added to a mayenite powder, and the resultant slurry is poured into a mold. The slurry is fixed onto the inside of the mold, and discharged. Alternatively, the contents of the mold may be solidified in the mold, to thereby form a compact. No particular limitation is imposed on the material of the mold. However, the mold is preferably formed of a ceramic material having the same composition as (or a composition similar to) that of a production target mayenite compound. When the mold is formed of a ceramic material having the same composition as (or a composition similar to) that of a production target mayenite compound, incorporation of impurities into a molded compact can be prevented. During formation of a compact, when the mold is pressurized, material fixation speed can be enhanced, leading to enhancement in productivity. In the case of forming a cylinder-shape compact, material fixation speed can be enhance through rotating the mold.

In one mode of potter's wheel molding, a solvent is added to a mayenite powder, and the resultant material is molded my means of a rotating machine such as a potter's wheel.

In one mode of thin sheet molding, a solvent is added to a mayenite powder, to thereby form a slurry, and the slurry is placed on a flat substrate. The slurry is formed into a thin sheet by means of a blade, while the thickness of the sheet is controlled. No particular limitation is imposed on the material of the substrate, and the mold may be formed from a ceramic material, a metallic material, or the like. Through this molding method, a molded product having a thin-film structure can be formed. A multi-layer structure may also be obtained through the following procedure. Specifically, the slurry applied onto the substrate is dried, and another (or the same) slurry is applied onto the dried slurry, followed by drying. The steps are repeated. Notably, after application of a slurry onto a substrate, drying of the slurry can be accelerated though hot air blowing, to thereby enhance productivity. The compact formed through the method may remain on the substrate and fired on the substrate, or may be removed from the substrate and fired.

Examples of the 3-dimensional laminating shaping method include ink jetting, powder lamination shaping, and slurry lamination shaping.

In one mode of ink jetting, a solvent, a binder, or the like is added to a mayenite powder, to thereby form a toner. The toner is fed to a 3D printer and injected through nozzles of the printer based on 3D data, to thereby form a compact having a shape of interest.

In one mode of powder lamination shaping, a powder mixture of a mayenite powder and a binder is fed to a molding cylinder so as to form stacked layers, and the mixture is heated through laser radiation such that the mixture is subjected to laser radiation to realize a pattern of interest based on the 3D data. As a result, the binder itself melts, to thereby binding the raw material powder via melting. Subsequently, the molding cylinder is lowered at a level equivalent to the corresponding thickness, and the powder mixture is again fed in a laminar manner. The newly fed raw material powder is melt-bound through laser radiation. Through repetition of the above procedure, a compact having a steric structure of interest can be obtained.

In one mode of slurry lamination shaping, a mayenite powder is dispersed in a liquid-form resin (photocruable or thermal curable rein), to thereby form a slurry, and the slurry is applied onto a substrate. The liquid resin is then cured through scanned laser radiation so as to have a pattern of interest. Thereafter, the slurry is again applied onto the cured rein layer, and the liquid resin is cured though laser radiation. Through repetition of the above procedure, a compact having a steric structure of interest can be obtained.

The compact formed through the 3-dimensional laminating shaping method can directly provide a target product of the mayenite compound I or the mixture II, in some cases of the type of the mayenite compound in powder form. If required, a step of heating the product in a reducing atmosphere, a step of heating the product in a sealed carbon crucible, and a step of irradiating the product with a radiation such as UV-ray, X-ray, or electron beam may be carried out. Through the aforementioned procedures, a mayenite compound enclosing an active species of interest can be yielded. Also, through this molding method, a variety of compacts having a complex shape can be produced.

[Second firing step] The powder-form or granular-form mayenite compound precursor, or a compact after the molding step, is placed in a crucible formed of an appropriate material, such as a noble metal (e.g., iridium) crucible or an alumina crucible, and the crucible is closed. In one mode, hydrogen gas is charged into a noble metal crucible, or the atmosphere of the crucible is changed to a reducing atmosphere by use of hydrogen gas or the like. The precursor or the compact is fired at 700°C or higher, whereby an oxygen element-containing enclosed anion can be substituted by H⁻.

In another mode, the mayenite compound precursor is placed in a carbon crucible, and the crucible is closed. An inert gas (e.g., nitrogen gas) is charged into the carbon crucible, or the atmosphere of the crucible is changed to an inert gas (e.g., nitrogen) atmosphere to vacuum. The precursor is fired at 700°C or higher, whereby an enclosed anion can be substituted by electron. In the case of the powder-form or granular-form mayenite compound precursor, a mixture thereof with carbon micropowder serving as a reducing agent may be fired. In the case where the mayenite compound precursor is confined in the carbon crucible and fired, the precursor more readily forms an electron-enclosing mayenite compound at a site in contact with the carbon crucible, while difficulty is encountered in formation of an electron-enclosing mayenite compound from the precursor at a site in no contact with the carbon crucible. Thus, in this case, uniformity in the product may decrease. In contrast, when a mixture of carbon micropowder with the mayenite compound precursor is fired, the contact area between carbon and the mayenite compound precursor increases, thereby attaining uniform contact. As a result, a uniform electron-enclosing mayenite compound can be yielded.

When the mixture of carbon and the mayenite compound precursor is molded and then fired, carbon contained in the contact is removed during firing to form CO gas and CO₂ gas, whereby a porous body of the mayenite compound can be produced. When the mixture of carbon and the mayenite compound precursor is fired in an inert gas (e.g., nitrogen) atmosphere or in vacuum, a porous body formed of an electron-enclosing mayenite compound can be produced. In the case where the mixture of carbon and the mayenite compound precursor is fired in air or an oxygen-containing atmosphere, the porous body formed via removal of CO gas and CO₂ gas is oxidized, and the mayenite compound encloses an anion such as active oxygen. Thus, when this porous body is fired in a carbon crucible under inert gas atmosphere (e.g., nitrogen) or in vacuum, a porous body formed of an electron-enclosing mayenite compound is yielded.

Meanwhile, in the case where a mixture of the powder-form or granular-form mayenite compound precursor with carbon micropowder serving as a reducing agent is fired under inert gas atmosphere (e.g., nitrogen) or in vacuum, substitution, by electron, of an anion such as active oxygen enclosed in a crystal cage cannot fully be attained under carbon-deficient conditions. In this case, active oxygen remains in crystal cages. When the amount of carbon to be mixed is excessive, carbon may remain. In the case where active oxygen remains in crystal cages due to carbon-deficient conditions, such active oxygen remaining in crystal cages can be substituted by electron through firing again in a carbon crucible under inert gas atmosphere or in vacuum. In the case where carbon may remain in the fired body due to carbon-excessive conditions, carbon can be readily removed from the mayenite compound after firing, through use of ball-form or pellet-form carbon (i.e., carbon particles having a size greater than that of the powder-form or granular-form mayenite compound precursor) instead of carbon micropowder.

No particular limitation is imposed on the upper limit of the heating temperature employed in a noble metal crucible or a carbon crucible. For example, the heating temperature is preferably 1,450°C or lower. Since the melting point of C12A7 is about 1,450°C, the mayenite compound precursor melts during heating at 1,450°C or lower, to thereby prevent change in form of the mayenite compound precursor. In the case where a variety of forms of the mayenite compound products are yielded via melting the mayenite compound precursor during heating, the heating temperature may be elevated to be higher than 1,450°C.

The longer the thermal reaction time in the carbon crucible or the noble metal crucible, the greater the substitution amount of oxygen-containing anion by electron or H⁻. In one specific case, when the mayenite compound precursor is heated in a noble metal crucible filled with hydrogen gas at 700°C for 240 hours, almost all enclosed oxygen ions can be substituted by H⁻.

The mayenite compound I may be produced through the following procedure. Specifically, a mayenite compound precursor in which oxygen ions are entirely or partially substituted by H⁻ is irradiated with a UV ray, an X ray, or an electron beam, to thereby partially substitute enclosed H⁻ by electron, whereby the mayenite compound I enclosing both electron and H⁻ is yielded. The wavelength of the UV ray exposed to the mayenite compound precursor may be adjusted to, for example, 250 nm to 350 nm. Alternatively, the mayenite compound I enclosing both electron and H⁻ can be also formed by confining the mayenite compound precursor in a carbon crucible and heating in a reducing atmosphere at 700°C or higher for a certain period of time.

When the mayenite compound I contains Ca, Al, and O particularly in the crystal lattice, the color tone of the compound varies in accordance with the enclosed electron or H⁻ content. Specifically, as the enclosed electron content increases, the mayenite compound becomes black, whereas as the enclosed electron content decreases, the mayenite compound becomes pale black to white.

The mixture II is obtained by mixing an H⁻-enclosing mayenite compound produced through heating in a reducing atmosphere, with an electron-enclosing mayenite compound produced by heating in a carbon crucible and/or heating a mixture of a mayenite compound precursor and carbon micropowder serving as a reducing agent, at a target ratio.

No particular limitation is imposed on the method of heating the mayenite compound precursor in the second firing step, and any known heating method may be employed. Examples of the heating method include furnace firing, electromagnetic wave firing, and pressure sintering.

In furnace firing, a mayenite compound precursor is placed in a furnace such as an electric furnace or a gas furnace and heated. In the heating method, when a drier atmosphere in the furnace (i.e., at lower vapor partial pressure) or a more deoxygenated atmosphere (i.e., at lower oxygen partial pressure) is employed, the produced mayenite compound can enclose electron and/or H⁻ at high concentration. One method for drying the furnace (i.e., for realizing a dry atmosphere) is placing a dehydrating agent in the furnace or in a gas feed path to the furnace. One method for reducing the oxygen partial pressure in the furnace is placing a deoxygenating agent in the furnace or in a gas feed path to the furnace. Through such means, a microamount of oxygen contained in the atmosphere gas can be removed.

In electromagnetic wave firing, the target material is fired through irradiation with an electromagmetic wave. One example of electromagnetic wave firing is a micrwave firing method. In this mode, preferably, the mayenite compound precursor is placed in a carbon container made of a material which readily absorbs an electromagnetic wave or a similar container and heated in the container, whereby the contents can be heated through radiation heat for a short period of time. Alternatively, the above temperature elevation can be attained for a short period of time by mixing a mayenite compound precursor with a material which readily absorbs electromagnetic wave. As described above, through electromagnetic wave firing in a dry atmosphere or a deoxygenating atmosphere, a mayenite compound which encloses electron and/or H⁻ at high concentration can be produced.

In pressure sintering, the target material is fired under pressurized conditions. Examples of the pressure sintering method include hot press sintering and heat isostatic pressing (HIP) sintering. As mentioned above, in pressure sintering, the source material is fired in a dry or deoxygenated atmosphere, to thereby produce a mayenite compound which encloses electron and/or H⁻ at high concentration.

In the second firing step, the mayenite powder or a compact obtained by molding the mayenite power is fired by being placed in or on a firing tool such as a crucible, a firing dish, a firing sheath or a firing furnace, followed by firing. In this case, for the same reason as mentioned in the column "Method for producing a mayenite compound precursor enclosing oxygen element-containing anion," the firing tools are preferably formed of a ceramic material having the same composition as (or a composition similar to) that of a production target mayenite compound.

In this way, the mayenite compound I and the mixture II can be produced.

Notably, in the aforementioned production methods, a mayenite compound precursor is produced through the first firing step, including an optional molding step and production of the mayenite compound enclosing electron and/or H⁻ is completed by carrying out the second firing step. Alternatively, a mixture obtained by the mixing/pulverization step without performing the first firing step is subjected to the second firing step, including an optional molding step and firing in a firing atmosphere of interest, to thereby yield a mayenite compound enclosing electron, a mayenite compound enclosing H⁻, or the mayenite compound I.

In the case where a molded product of the mayenite compound I or the mixture II is produced, the molded product may be subjected to cutting, grinding, polishing, or the like, to thereby adjust the form and dimensions thereof to values of interest. In this case, grinding or a similar working is performed by use of water or oil, in some cases. Since a mayenite compound enclosing electron and a mayenite compound enclosing H⁻ may react with water or oil, the processing procedure is performed under dry conditions or by use of a liquid with high stability. Through tuning the working atmosphere (i.e., selecting an inert atmosphere or a reducing atmosphere), oxidation of the mayenite compound I and the mixture II can be prevented.

In the case where the mayenite-compound-containing product is formed only of the mayenite compound I or the mixture II, the aforementioned production methods may be employed. In the case where the mayenite-compound-containing product is formed of the material containing the mayenite compound I or the mixture II, and an additional substance, the aforementioned substances employed in the aforementioned steps may be incorporated thereinto. In a specific mode, a mayenite compound precursor is mixed with a ceramic power or the like, to thereby form a compact, and the compact is fired. Alternatively, a powder mixture of the mayenite compound I or the mixture II with an additional ceramic powder is molded to form a compact.

[Method for producing porous body] The porous body of the mayenite compound I or the mixture II may be produced through a solvent vaporization technique, an acid corrosion technique, a hydration technique, etc.

In a solvent vaporization technique, a mayenite powder is mixed with a binder such as organic solvent, water, or resin, and the mixture is molded, followed by calcining to remove the binder via vaporization, whereby a porous body is formed. Pores are provided through removal of the binder. Through appropriately tuning the type, amount, etc. of the binder, the porosity, pore size, specific surface area, etc. of the porous body can be modified.

In an acid corrosion technique, a mayenite powder or compact is immersed in an acid such as sulfuric acid or nitric acid, to thereby corrode at least the surface thereof. As a result, a porous body having at least a porous surface is yielded. Through appropriately tuning the type, concentration, pH, etc. of the acid, the porosity, pore size, specific surface area, etc. of the porous body can be modified.

In a hydration technique, a mayenite powder or compact is brought into contact with water, to thereby induce hydration reaction at least on the surface thereof. As a result, the crystalline surface is broken, to thereby yield a porous body having at least a porous surface. Through appropriately tuning the amount, temperature, etc. of water to be brought into contact with the mayenite powder or compact, hydration can be controlled, whereby the porosity, pore size, specific surface area, etc. of the porous body can be modified.

[Method for producing coating film] A coating film containing the mayenite compound I or the mixture II can be formed on a target object through a method such as a coating technique, a thermal spraying technique, or a glazing technique.

In the coating technique, a resin, a solvent, or the like is added to a mayenite powder, to thereby prepare a coating material. The coating material is appropriately applied onto an object and dried, whereby a coating film is formed on the surface of the object.

In a thermal spraying technique, a mayenite powder is heated, and droplets of the molten spraying material are sprayed to an object by means of high-flow-speed gas or the like, to thereby form a coating film on the surface of the object. Through employing a reducing gas atmosphere in the thermal spraying step, H⁻ can be enclosed in inclusion cages. Also, when the object on which a coating film has been formed through thermal spraying is fired in an appropriate atmosphere, the active species enclosed in inclusion cages can be selected.

In a glazing technique, a mayenite powder is suspended in an organic solvent (e.g., alcohol), water, or other solvents, to thereby prepare a glaze, and the glaze is appropriately applied onto the surface of an object, followed by firing, to thereby form a glassy coating film on the surface of the object. In this technique, the glaze may be prepared from not only a mayenite powder but also be a suspension formed by suspending in a solvent a powder mixture of a Ca-containing compound and an Al-containing compound. Through applying a glaze having a specific Ca/Al ratio onto an appropriate surface of an object and firing, a coating film of a mayenite compound can be formed. When a reducing gas atmosphere is employed in firing, a coating film formed of a mayenite compound enclosing H⁻ can be produced.

### Method for producing filter

A filter made of the mayenite compound I or the mixture II can be produced through, for example, the following procedures. In one procedure, a nonwoven fabric piece or a fabric piece, which is formed from natural fiber or chemical fiber through a known method, is immersed in a solution in which a powder of the mayenite compound I or mixture II is dispersed, to thereby deposit the mayenite compound I or the mixture II on the nonwoven fabric piece or the fabric piece. In another procedure, the mayenite compound I or the mixture II is deposited on natural fiber or chemical fiber, and nonwoven fabric or fabric is formed from such a fiber, to thereby produce a filter having a shape of interest. In still another procedure, a porous filter is fabricated in a manner similar to that employed in producing the porous body of the mayenite compound I or the mixture II. In still another procedure, a filter is produced by filling a specific space with a powder-form or granular-form multifunctional agent.

### Method for glass material

The mayenite compound encloses an active species such as electron, H⁻, or the like, even in a molten state. Thus, when the molten-state is quenched, the compound in glass state can be formed. That is, a glass material formed of the mayenite compound I or the mixture II can be produced via melting and quenching. By use of a mold having a shape of interest in quenching, a glass material of the mayenite compound having the corresponding shape can be produced. Examples of the material of the mold include a ceramic material, a metal, and carbon. In the case of producing a glass plate, a molten mayenite compound precursor is poured onto molten tin. Since the mayenite compound enclosing electron and the mayenite compound enclosing H⁻ oxidize through contact with oxygen present in air, forming of glass thereof is preferably performed in an inert atmosphere(e.g., N₂ or Ar), under hydrogen, or in vacuum. Any of glass forming techniques conventionally employed in the art may be employed, such as blowing, blow molding, press molding, flashing, casting, hot casting, cold casting, and sand casting. Also, through wire drawing a molten mayenite compound, fiber of the compound can be yielded.

### Multifunctional agent

The multifunctional agent according to the present invention will next be described in detail.

The multifunctional agent of the present invention contains at least one of the mayenite compound I enclosing electron and H , and a mixture II of the mayenite compound enclosing electron and the mayenite compound enclosing H⁻. More specifically, the multifunctional agent may contain only the mayenite compound I or only the mixture II. Hereinafter, the mayenite compound I or the mixture II contained in the multifunctional agent may be referred to as simply "mayenite compound."

The mayenite compound I or the mixture II has both a function of a mayenite compound enclosing only electron and a function of a mayenite compound enclosing only H⁻.

The mayenite compound enclosing electron has electron donating property, to thereby scavenge free radicals, in particular active oxygen, and reducing property.

The mayenite compound enclosing H⁻ has H⁻ donating property and reducing property.

The mayenite compound has a cage-like crystal structure, where at least one of electron and H⁻ (negatively charged species) is enclosed in a positively charged inclusion cage. The mayenite compound I, having a crystal lattice including at least Ca, Al, and O, can incorporate anions, in particular oxygen ions, into the inclusion cages and can remove water present around the cages via hydration reaction via contact with water. When oxygen or water is present around the mayenite compound I, firstly, the compound I incorporates oxygen ions O²⁻ into inclusion cages. Then, the incorporated oxygen ions O²⁻ react with water around the compound I, to thereby form OH⁻ in the cages. In this way, the mayenite compound I is involved in deoxygenation reaction and dehydration reaction.

Thus, the mayenite compound I or the mixture II exhibits multifunctions: radical scavenging activity and reducing property (by electron donating property); reducing property (by H⁻ donating property); dehydration performance (through hydrogenation reaction via contact with water); and deoxygenation performance (by incorporating oxygen ions and the like into the inclusion cage).

In the multifunctional agent according to the present invention, no particular limitation is imposed on the ratio in amount of electron to H⁻, two components being enclosed in the mayenite compound I. In the mixture II, no particular limitation is imposed on the ratio in amount of the mayenite compound enclosing electron and that enclosing H⁻. These ratios may be controlled in accordance with use of the multifunctional agent. So long as the aforementioned functions are ensured, the multifunctional agent according to the present invention may further contain a mayenite compound other than the mayenite compound I and the mixture II. Examples of such additional compounds include a mayenite compound enclosing an ion other than electron and H⁻ and a mayenite compound in which Al and Ca forming the inclusion cages are partially substituted by another element.

Hereinafter, the functions of the multifunctional agent will next be described in detail.

The multifunctional agent is insoluble in any solvent. The solvent may be a hydrophilic solvent or a hydrophobic solvent. A preferred example of the hydrophilic solvent is water, and a preferred example of the hydrophobic solvent is an organic solvent such as oil. Thus, the multifunctional agent according to the present invention is insoluble in water or oil.

In use of the multifunctional agent, the mayenite compound serving as a component substance donates electrons to molecules (other than the mayenite compound), ions, radicals, etc. More specifically, in the multifunctional agent, the following reaction may occur. That is, electrons enclosed in the mayenite compound are directly transferred to molecules (other than the mayenite compound), ions, radicals, etc., which are present outside the cage cluster. When electrons enclosed in the mayenite compound are directly transferred to radicals which are present outside the cage cluster, highly reactive radicals are scavenged, whereby proceeding of radical chain reaction can be prevented. In one specific mode, by placing the multifunctional agent in a space where active oxygen is present, electrons are transferred to active oxygen, to thereby scavenge active oxygen. Thus, oxidation can be stopped. In another specific mode, electrons enclosed in the mayenite compound are transferred to benzene rings, to thereby cause Birch reduction. Thus, hazardous substances can be detoxified. Also, in use of the multifunctional agent, the following reaction may occur. That is, H⁻ enclosed in the mayenite compound is directly transferred to molecules (other than the mayenite compound), ions, etc., which are present outside the cage cluster. In one specific mode, when H⁻ enclosed in the mayenite compound is transferred to a carbonyl compound, hydride reduction occurs, to thereby reduce an aldehyde and a ketone to alcohols. Thus, hazardous substances can be detoxified.

In the multifunctional agent, the mayenite compound serving as a component substance has a cage-like crystal structure, which induces hydration reaction via contact with water, thereby forming a hydrate. Thus, the multifunctional agent exhibits dehydration performance. Once the hydration occurs, crystal cages are broken, and enclosed active species (e.g., electron and H⁻) are released from the cages. As the hydration proceeds, not only crystal cages at the surface of the cage cluster but also those inside the cage cluster are broken. As a result, enclosed active species present inside the crystal cages can be involved in the reaction. Thus, when the multifunctional agent is used in the presence of water, the activity of the multifunctional agent can be maintained for a long period of time.

### Use of multifunctional agent

The multifunctional agent can find a variety of uses by virtue of its various functions. For example, the multifunctional agent may be used in a liquid such as a solvent, while it is statically placed. The multifunctional agent is not dissolved in any solvent and retains its solid state in liquid. Even being not dissolved in a solvent, the multifunctional agent transfers electrons to molecules forming the solvent, ion, radicals, etc. which are dissolved in the solvent, or molecules, ions, etc. which are dispersed in the solvent, thereby causing reduction of such species.

Hereinafter, the multifunctional agent for use in oil, which is a typical example of the hydrophobic solvent, will be described. The oil may be a liquid which contains an organic compound and which does not intermingle with water. Examples of the oil include mineral oil, vegetable oil, and animal oil. Specific examples of these oils include insulating oil, petroleum, kerosene, light oil, food oil, lubricating oil, engine oil, silicone oil, essential oil, perfumed oil, hair oil, biofuels, organic coatings containing drying oil, fish oil, lard, whale oil, horse oil, wax, and waste oils thereof.

No particular limitation is imposed on the form and mode of the multifunctional agent, so long as it is a solid insoluble in liquid (e.g., oil). Examples of the form of the multifunctional agent include powder, granule, ball, filter, coating film, porous body, and container.

Specific uses of the multifunctional agent for use in oil will next be described.

The multifunctional agent may be used for maintaining the quality of oil. The multifunctional agent serves as an antioxidant, a reducing agent, or a dehydrating agent. Specifically, the radical scavenging activity and deoxygenation performance of the multifunctional agent lead to prevention of oxidation of oil; the reducing property thereof leads to regeneration of oxidized oil; and the dehydration performance thereof leads to removal of water from oil, to thereby prevent deterioration of the oil.

The multifunctional agent also serves as an antioxidant. When being allowed to stand in oil, oxidation of hydrocarbons serving as oil components can be prevented, to thereby suppress deterioration of the oil. In the case where oxygen radical species such as superoxide anion radicals and hydroxyl radicals are present in oil, hydrocarbons in the oil are readily oxidized. When the multifunctional agent is settled in oil, electrons enclosed in the mayenite compound are transferred to oxygen radicals, to thereby scavenge oxygen radicals, leading to prevention of oxidation of hydrocarbons. Also, oxygen, oxygen ions, and the like present outside the inclusion cage are substituted by ions or the like enclosed in the inclusion cage, whereby oxygen ions in the inclusion cage are trapped. As a result, the dissolved oxygen ion content of the oil can be reduced, to thereby prevent oxidation of hydrocarbons serving as oil components.

The multifunctional agent also serves as a reducing agent. When being in contact with oil, an oxidized-form hydrocarbon, in which oxidation has proceeded, is reduced, to thereby regenerate a reduced-form hydrocarbon. When the multifunctional agent is used as a reducing agent, the quality of oil deteriorated due to oxidation of the hydrocarbon can be improved. The reducing agent can reduce a hydrocarbon, when the agent donates electrons enclosed in the mayenite compound to the oxidized hydrocarbon present in oil. In addition, as a result of oxidation of hydrocarbon, a carbonyl compound is formed. The reducing agent feeds H⁻ enclosed in the mayenite compound to the carbonyl compound, to thereby induce hydride reduction. As a result, the carbonyl compound is reduced to a corresponding alcohol.

The multifunctional agent also serves as a dehydrating agent and can remove water from oil through contact with oil. The mayenite compound serving as a component substance of the multifunctional agent has a cage-like crystal structure, which induces hydration reaction via contact with water, thereby forming a hydrate. Thus, water can be removed from oil. Through hydration reaction, crystal cages are broken, and enclosed species (e.g., electron and H⁻) are released from the cages. As the hydration proceeds, not only crystal cages at the surface of the cage cluster but also those inside the cage cluster are broken. As a result, enclosed electron and H⁻ present inside the crystal cages can be involved in prevention of deterioration of oil.

One specific embodiment of the multifunctional agent is a granular-form multifunctional agent as shown in FIG. 2. The granular-form multifunctional agent contains a mayenite compound and an optional ingredient. The particle size of the granules of the mayenite compound contained in the multifunctional agent is preferably 0.1 mm to 2.0 mm (not including 2.0 mm). In order to prevent diffusion of granules of the multifunctional agent in oil, the granular-form multifunctional agent is placed in a bag 7 as shown in FIG. 2. The bag 7 is oil-permeable but has an opening having such a size that the granular-form multifunctional agent and a hydrate held therein cannot leak to the outside. An example of the bag is a bag having a mesh structure. According to the embodiment shown in FIG. 2, the bag 7 containing the granular-form multifunctional agent 8 is sunk in a container 10 where oil 9 is placed. In order to facilitate removal of the bag 7 from the container 10, the bag 7 may be a bag with a string, and one end of the string may be placed outside the container 10. In a transfer image of the inside of the bag 7 shown in FIG. 2 (right side), a white circle denotes a substance present in the oil, such as oxygen ions or oxidized hydrocarbon, while a black circle denotes a substance enclosed in and trapped by inclusion cages of the mayenite compound, such as oxygen ions or reduced hydrocarbon. Oxygen ions present in the oil are trapped by inclusion cages of the mayenite compound present in the bag, whereby the oxidizing performance is lost. Also, oxygen radicals present in the oil are scavenged by transferring thereto electrons of the mayenite compound present in the bag. Oxidized hydrocarbon present in the oil is reduced by electron or H⁻ enclosed in the mayenite compound, while water present in the oil reacts with the mayenite compound, to thereby form a hydrate. Through the above mechanism, oxidation of the hydrocarbon(s) in oil and deterioration of the oil can be prevented by placing the multifunctional agent accommodated in the bag. Meanwhile, another specific embodiment of the multifunctional agent is a powder-form multifunctional agent. The powder-form multifunctional agent has a particle size smaller than that of the granular-form multifunctional agent and assumes the form of a powder. Through charging the powder-form multifunctional agent into a bag as shown in FIG. 2, and immersing the bag in the target oil, oxidation of hydrocarbon in the oil can also be prevented. The bag 7 may be placed in the oil 9 as shown in FIG. 2, but may be placed in air around the oil 9. In the latter case, the multifunctional agent can remove water present around the oil, and oxygen ions are enclosed in the inclusion cages. Thus, the powder-form multifunctional agent also exhibits dehydration performance and deoxygenation performance.

The aforementioned granular-form multifunctional agent is suitably used as an antioxidant for food oil products such as salad oil, sesame oil, soybean oil, and rapeseed oil. In a specific manner, the granular-form multifunctional agent placed in the bag is caused to sink in a plastic container accommodating a target food oil. Generally, during long-term storage of food oil, oxidation of oil is accelerated by oxygen in air, moisture, heat, light, metal ions, microorganisms, etc., resulting in deterioration of oil (i.e., rancidity or spoilage). Particularly when an oil is allowed to stand in air for a long period time, an unsaturated fatty acid in the oil absorbs oxygen, to thereby form a superoxide, which is a peroxide with poor stability. The superoxide is rearranged to form an unsaturated hydroperoxide, which accelerates oxidation of oil. Since food oil contains large amounts of unsaturated fatty acids such as oleic acid, linoleic acid, and linolenic acid, food oil is highly susceptible to oxidation reaction, to thereby generate lipid peroxide. Lipid peroxide accelerates oxidation reaction in the human body, to thereby provide carcinogenicity. Thus, from the viewpoint of health, oxidation of unsaturated fatty acid in food oil is desirably prevented.

When the granular-form or powder-form multifunctional agent is placed in a food oil, radicals present in the food oil can be scavenged. In a more specific scavenging mechanism, electrons in the inclusion cages of the mayenite compound are transferred to the radicals contained in the food oil. For example, hydroxyl radicals ·OH present in the food oil can be scavenged by electrons in the inclusion cages transferred to the radicals, to thereby form hydroxide ions OH⁻, having less oxidation performance.

By use of the multifunctional agent, oxygen dissolved in a target food oil is enclosed in the mayenite compound, to thereby lower the dissolved oxygen level of the food oil. As a result, growth of aerobic bacteria can be prevented in the food oil, thereby attaining a longer-term storage of the food oil.

As described above, through immersing the granular-form multifunctional agent in a target food oil, generation of a peroxide hazardous to the human body, which would otherwise be caused by progress of oxidation reaction of hydrocarbon in the oil over time, can be prevented. By use of the multifunctional agent, deterioration of food oil can be prevented, to thereby prolong the life of the food oil.

The granular-form or powder-form multifunctional agent is not dissolved in oil. Thus, when the multifunctional agent is allowed to stand in fool oil, dissolution of a component hazardous to the human body is prevented. Also, the multifunctional agent does not modify the components of the food oil, and is not restricted by food-additive-level-related regulations. Furthermore, even when a large amount of the multifunctional agent is added to a food oil, the agent does not affect the components of the food oil. Thus, a desired amount of the multifunctional agent can be added to the food oil. As a result, a required amount of the multifunctional agent can be added to the food oil, to thereby satisfactorily prevent oil deterioration. Meanwhile, the multifunctional agent can fully provide a function as an antioxidant in a high-temperature range (to about 300°C). Vitamin E, which is generally added to food oil as an antioxidant, has poor heat resistance, and the function thereof as an antioxidant is reduced at high temperature. In contrast, the multifunctional agent of the present invention can exhibit a function of an antioxidant even at high temperature. Thus, a bag including the multifunctional agent and food oil, placed simultaneously in a cooking apparatus (e.g., a pan or a fryer), can be subjected to cooking by heat. Through adding a bag including the multifunctional agent, as an antioxidant, to unoxidized oil under cooking, the life of the food oil can be prolonged.

The granular-form or powder-form multifunctional agent may be used for maintaining the quality of oils other than food oil. The granular-form or powder-form multifunctional agent as shown in FIG. 2 is used in, for example, a container for essential oil (also called aroma oil); a cosmetic container for cosmetic oil (e.g., perfumed oil); a fuel tank of a four-wheel automobile or a motorcycle (more specifically, a car or a bike), an agricultural machine, a power generator, a vehicle employing an internal combustion engine (e.g., carts), an aircraft, a ship, etc.; an oil tank of a stove or the like, a plastic tank for oil storage, and a large-scale metal oil tank for a house (also called a "home tank"); a tank for oil reservation; a transformer tank for oil-immersed transformers; a capacitor tank for oil-impregnated capacitors; and the like. In an actual procedure, the granular-form or powder-form multifunctional agent placed in a bag is immersed in a target tank or container, whereby deterioration of oil placed in the container or tank can be prevented.

The granular-form or powder-form multifunctional agent is suitably used as a multifunctional agent for use in insulating oil, which agent is provided for maintaining insulating oil. The multifunctional agent for use in insulating oil is employed for maintaining the quality of an insulating oil used in, for example, an oil-immersed transformer, an oil-impregnated capacitor, an oil-impregnated cable, an oil-impregnated breaker. etc. Such an insulating oil is defined by JIS C 2320, and examples thereof include those based on mineral oil, alkylbenzene, polybutene, alkylnaphthalene, alkyldiphenylalkane, silicone oil, etc.

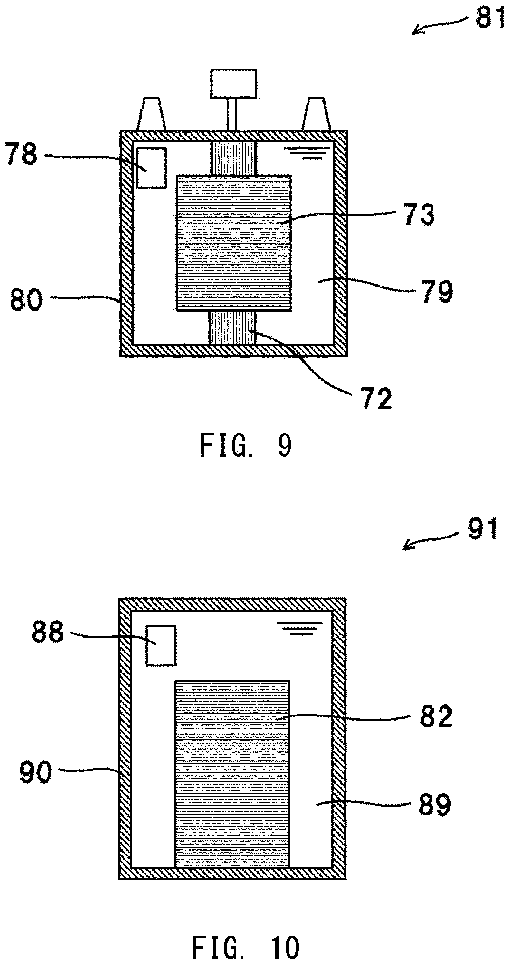

FIG. 9 is a sketch of an example of the oil-immersed transformer. An oil-immersed transformer 81 has an iron core 72, a coil 73 wound around the iron core 72, a transformer tank 80 accommodating the iron core 72 and the coil 73, an insulating oil 79 charged in the transformer tank 80, and a bag 78 including a multifunctional agent and sunk in the insulating oil 79. The insulating oil 79 is gradually oxidized over time by the effects of temperature, oxygen, water, etc., to thereby form a carbonyl compound and water. Generally, as the water content of the insulating oil 79 increases, corrosion tends to occur in the transformer tank 80 and the like. In such a case, sludge may form in the insulating oil 79, possibly causing breakdown. In contrast, in the oil-immersed transformer 81 of the embodiment, a bag including a granular-form or powder-form multifunctional agent in a mesh bag 78 is sunk in the insulating oil 79. Thus, oxygen radicals present in the insulating oil 79 are scavenged by transferring electrons from the multifunctional agent. Also, oxygen ions present in the insulating oil 79 are trapped by inclusion cages of the mayenite compound. As a result, the insulating oil 79 in which the bag 78 including the multifunctional agent has been immersed becomes resistive to oxidation. Meanwhile, an oxidized hydrocarbon present in the insulating oil 79 is reduced by electron or H⁻ enclosed in the mayenite compound, to thereby regenerate a reduced-form hydrocarbon. Therefore, the insulating oil 79 in which the bag 78 including the multifunctional agent has been sunk can be modified to recover its quality, even the hydrocarbon has been oxidized. In addition, when the bag 78 including the multifunctional agent is present in the insulating oil 79, water present in the insulating oil 79 reacts via hydration with the mayenite compound contained in the multifunctional agent, to thereby form a hydrate. The thus-formed hydrate is accumulated in the bag, to thereby remove water from the insulating oil 79. As a result, deterioration of the insulating oil 79 can be prevented. In the case where the multifunctional agent is formed of a porous body, sludge present in the insulating oil 79 can be adsorbed.

Generally, the oil-immersed transformer 81 employs electrically insulating paper as a conductor coating of the coil 73. The electrically insulating paper is formed of craft pulp, Manila hemp, paper bush (*Mitsumata*), or the like, which is formed of cellulose as a main ingredient substance. When the electrically insulating paper formed of cellulose as a main ingredient substance is deteriorated, the electrically insulating paper is chemically changed, to thereby form an alcohol. Then, from the alcohol, an aldehyde, furfural, a carbonyl compound are sequentially formed. Finally, CO, CO₂, and water are formed. The electrically insulating paper may be broken through deterioration. Once the electrically insulating paper is broken, breakdown may occur. In contrast, when the bag 78 including the multifunctional agent is present in the insulating oil 79, an aldehyde, a carbonyl compound, and the like are reduced by electron or H⁻ enclosed in the mayenite compound, whereby deterioration of the insulating oil is suppressed, to thereby maintain the quality of the insulating oil.

In the case where water is generated in the insulating oil 79 due to deterioration of the insulating oil 79 and the insulating paper, the mayenite compound of the multifunctional agent is hydrated with water, to thereby form a hydrate. As a result, water in the insulating oil 79 can be removed. In addition, the mayenite compound of the multifunctional agent is hydrated with water in the insulating oil 79, whereby crystal cages are broken, and enclosed active species (e.g., electron and H⁻) are released from the cages. As the hydration proceeds, not only crystal cages at the surface of the cage cluster but also those inside the cage cluster are broken. As a result, enclosed active species present inside the crystal cages can be involved in the reaction. Thus, the function of the multifunctional agent can be ensured for a long period of time.

In the above embodiment, the bag 78 including granular-form or powder-form multifunctional agent is immersed in the insulating oil 79. However, no particular limitation is imposed on the embodiment of the multifunctional agent. Examples of the embodiment include an embodiment in which a ball-form or pellet-form multifunctional agent not accommodated in a bag or the like is immersed in insulating oil; an embodiment in which the inner surface of the transformer tank 80 is coated with a coating film containing a multifunctional agent; and an embodiment in which a material for forming the transformer tank 80 contains a multifunctional agent.

In the case of an oil-immersed transformer 81 having a partially opened (non-closed type) transformer tank 80 as shown in FIG. 9, the insulating oil 79 is exposed to air. The location of the multifunctional agent is not limited to the insulating oil 79, and may be placed around the insulating oil 79; i.e., in air in contact with the interface of the insulating oil 79. Similar to the case where the multifunctional agent is placed in the insulating oil 79, when the multifunctional agent is placed around the insulating oil 79, oxygen radicals present in air in the vicinity of the insulating oil 79 are scavenged, and oxygen ions are incorporated into inclusion cages, whereby oxidation of the insulating oil 79 is prevented. In addition, water contained in air in the vicinity of the insulating oil 79 is removed by the multifunctional agent, to thereby prevent deterioration of the insulating oil 79. Thus, the multifunctional agent placed around the insulating oil 79 (e.g., at an air intake opening of the transformer tank 80) may be used as a substitute of the dehydrating agent/deoxygenating agent, to thereby prevent deterioration of the insulating oil 79.

FIG. 10 is a sketch of an example of the oil-impregnated capacitor, an oil-impregnated capacitor 91. As shown in FIG. 10, the capacitor 91 has a capacitor 82 accommodating a plurality of capacitor elements, a capacitor tank 90 accommodating the capacitor 82, an insulating oil 89 charged in the capacitor tank 90, and a bag 88 including a multifunctional agent and immersed in the insulating oil 89. Similar to the oil-immersed transformer 81, the oil-impregnated capacitor 91 has the bag 88 including a multifunctional agent in the insulating oil 89. Thus, oxidation of the insulating oil 89 is prevented, and, if deteriorated, the insulating oil 89 is refreshed. In addition, water in the insulating oil 89 is removed. No particular limitation is imposed on the form of the multifunctional agent employed in the oil-impregnated capacitor 91. Similar to the case of the oil-immersed transformer 81, the multifunctional agent may assume the form of ball, pellet, coating film, etc. In the case of the oil-impregnated capacitor 91 having a partially opened (non-closed type) capacitor tank 90 as shown in FIG. 10, the insulating oil 89 is exposed to air. The location of the multifunctional agent is not limited to the insulating oil 89, and the agent may be placed around the insulating oil 89, similar to the oil-immersed transformer 81. Thus, the multifunctional agent placed around the insulating oil 79 (e.g., at an air intake opening of the capacitor tank 90) may be used as a substitute of the dehydrating agent/deoxygenating agent.

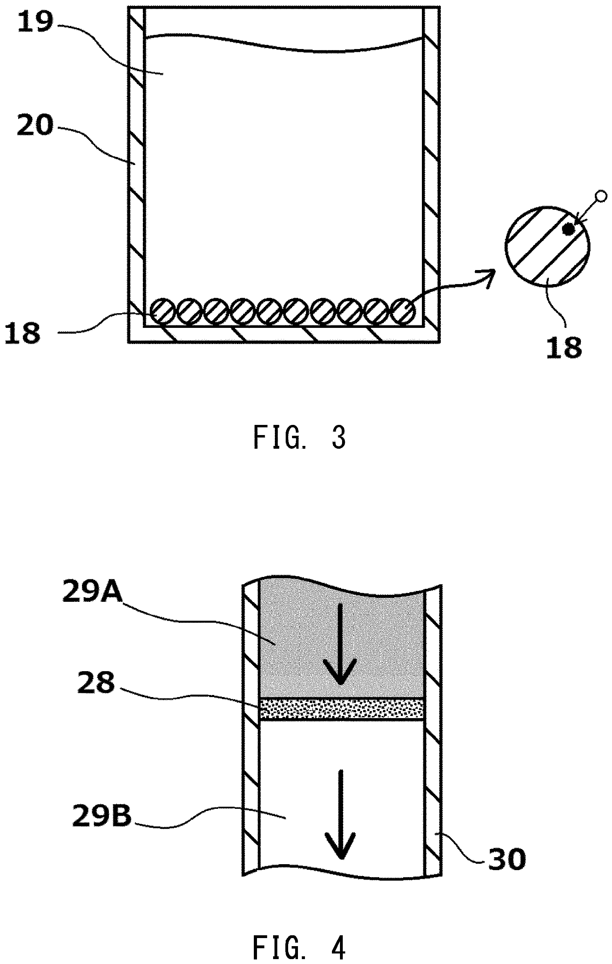

Another embodiment of the multifunctional agent is a ball-form multifunctional agent 18 as shown in FIG. 3. The ball-form multifunctional agent can be produced by adding an appropriate solvent, binder, or the like to a powder-form mayenite compound and molding the resultant mixture to a size, shape, or the like of interest. Generally, the ball-form multifunctional agent is greater in size than the aforementioned granular-form multifunctional agent. Preferably, the ball-form multifunctional agent is generally spherical, and the size of the sphere (i.e., particle size) is 2 mm to 50 mm. So long as the effects of the present invention are ensured, the ball-form multifunctional agent may further contain an ingredient other than the mayenite compound. Meanwhile, a specific example of the multifunctional agent having a shape other than the generally spherical shape is a pellet-form multifunctional agent. So long as the shape of the pellet-form multifunctional agent is not a generally spherical shape, the shape thereof may be generally columnar, generally prismatic, generally truncated conical, frustum of generally polygonal pyramid, etc. The aforementioned ball-form or pellet-form multifunctional agent may be a dense body or a porous body. Through modifying the pore size, pore density, etc. of the ball-form or pellet-form multifunctional agent, the specific surface area and the like of the multifunctional agent can be tuned. The pellet-form multifunctional agent may be used in the same applications as those described in relation to the ball-form multifunctional agent, in the specification.

In FIG. 3, the ball-form multifunctional agent 18 is sunk into the container 20 accommodating oil 19 and allowed to stand the bottom of the container. In an enlarged view of the ball-form multifunctional agent 18 shown in FIG. 3 (right side), a white circle denotes a substance present in the oil, such as oxygen ions, oxygen radicals, oxidized hydrocarbon, and the like. In the aforementioned transfer image of the inside of the bag 7 shown in FIG. 2 (right side), a black circle denotes a substance enclosed in and trapped by inclusion cages of the mayenite compound, such as oxygen ions or reduced hydrocarbon. Oxygen ions present in the oil are trapped by inclusion cages of the mayenite compound present, whereby the oxidizing performance is lost. Also, oxygen radicals present in the oil are scavenged by transferring thereto electrons of the mayenite compound. Oxidized hydrocarbon present in the oil is reduced by electron or H⁻ enclosed in the mayenite compound, while water present in the oil reacts with the mayenite compound, to thereby form a hydrate. Thus, by placing the ball-form multifunctional agent into a target oil, oxidation of hydrocarbon in the oil can be prevented, whereby deterioration of the oil can be suppressed.

The ball-form multifunctional agent is suitably used in, for example, an oil reservation tank. Specifically, the ball-form multifunctional agent may be placed at the bottom of a large-scale tank for oil reservation so as to cover the bottom surface. During long-term storage, oxidation of hydrocarbon in the oil (petroleum) proceeds, resulting in deterioration of the oil. More specifically, the color tone of the petroleum changes by reaction with oxygen, and a gummy blackish brown substance may generate in some cases. In the oxidation of hydrocarbon, hydrogen is extracted from a hydrocarbon contained in the petroleum, and the extraction site serves as a starting point of the oxidation. The hydrogen-extracted carbon atoms react with oxygen molecules, to thereby generate a superoxide. Through progress of radical chain reaction by the superoxide, oxidation of hydrocarbon in the oil is accelerated. Therefore, there is prevented deterioration of oil (petroleum), causing deterioration of petroleum fuel, change in color tone of oil, etc.

Through immersion of the ball-form multifunctional agent in an oil reservation tank, oxidation of hydrocarbon in the oil is prevented. More specifically, electrons enclosed in the mayenite compound are transferred to radicals generated in the oil, to thereby generate ions from the radicals. As a result, the radicals can be scavenged. The mayenite compound donates electrons to the superoxide generated in the step of oxidation of hydrocarbon, whereby the superoxide is decomposed, and oxidation of hydrocarbon can be suppressed. Thus, by use of the ball-form multifunctional agent of the present invention, oxidation of hydrocarbon in the oil reserved in an oil reservation tank can be prevented, to thereby realize long-term reservation of oil without deterioration of the quality of the oil in reservation.

The ball-form multifunctional agent is not dissolved in oil (petroleum). Thus, the multifunctional agent does not modify the components of the oil, and is not restricted by regulations regarding oil additive level. Furthermore, even when the large amount of the multifunctional agent is added to an oil, the agent does not affect the components of the petroleum oil. Thus, a desired amount of the multifunctional agent can be added to the oil. As a result, a required amount of the multifunctional agent can be added to the oil, to thereby satisfactorily prevent oil deterioration. Thus, longer-term reservation of oil is realized without deterioration of the quality of the oil in reservation.

The aforementioned ball-form multifunctional agent may also be employed in containers other than an oil reservation tank. Specifically, the ball-form multifunctional agent may be sunk into an oil tank for storage of oil (kerosene) or placed in a tank lorry, a tanker, etc. for transporting fuel oil (e.g., petroleum). Alternatively, the ball-form multifunctional agent may be sunk into a container for accommodating essential oil, perfumed oil, food oil, etc. By use of the ball-form multifunctional agent of the present invention, oxidation of oil contained in a tank, a tank lorry, a tanker, a container, etc. can be prevented. In another use of the ball-form multifunctional agent, the agent is directly added to a cooking apparatus (e.g., a pan or a fryer) upon cooking with oil by heat. Through addition of the ball-form multifunctional agent, oxidation of the oil used in the cooking apparatus can be retarded. In other words, through adding the ball-form multifunctional agent, as an antioxidant, to unoxidized oil under cooking, the life of the oil can be prolonged.

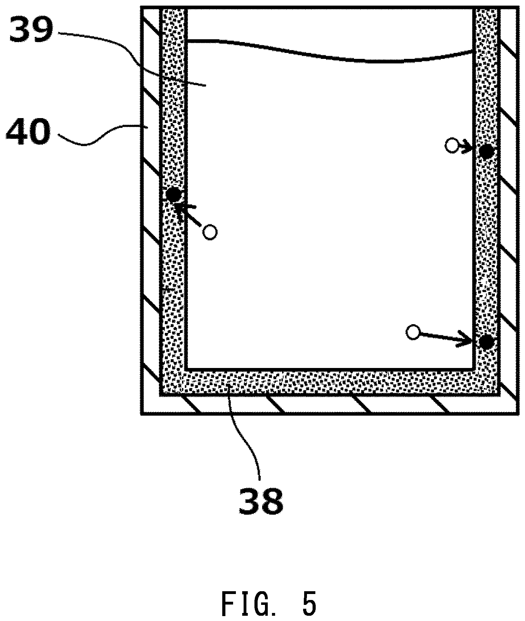

Another specific embodiment of the multifunctional agent is a coating film-shaped multifunctional agent 38 as shown in FIG. 5. The coating film-shaped multifunctional agent may be produced by preparing a slurry of a powder-form mayenite compound with an optional solvent, binder, etc., applying the slurry onto the inner wall surface of the container for storage of oil or the like, and solidifying the slurry. As shown in FIG. 5, at least a part of an oil 39 contained in a container 40 comes into contact with the coating film-shaped multifunctional agent 38 formed on the inner wall surface of the container 40. In FIG. 5, a white circle denotes a substance present in the oil, such as oxygen ions, oxygen radicals, or oxidized hydrocarbon, while a black circle denotes a substance trapped by inclusion cages of the mayenite compound, such as oxygen ions or reduced hydrocarbon. Oxygen ions present in the oil are trapped by inclusion cages of the mayenite compound present in the coating film-shaped multifunctional agent 38, whereby the oxidizing performance is lost. Also, oxygen radicals present in the oil are scavenged by transferring thereto electrons of the mayenite compound present in the multifunctional agent. Oxidized hydrocarbon present in the oil is reduced by electron or H⁻ enclosed in the mayenite compound. Thus, by storing a target oil or the like in a container having a coating film formed from the multifunctional agent, oxidation of hydrocarbon in the oil can be prevented, whereby deterioration of the oil can be suppressed.

The coating film-shaped multifunctional agent is suitably used for storing, for example, an organic coating such as ink or paint. In a specific procedure, a coating film of the multifunctional agent is formed on the inner wall surface of a can for accommodating the organic coating. Oxygen molecules entering from the outside of the can are trapped by the mayenite compound contained in the coating film-shaped multifunctional agent, whereby oxidation of the organic coating can be prevented. The coating film-shaped multifunctional agent is particularly suitably used for the storage of oil coloring paint. Oil coloring paint is an oily liquid containing a pigment and a drying oil. After application onto paper or the like, the drying oil contained in the oil coloring paint is oxidized, whereby the paint liquid is hardened. As a result, the coloring paint is fixed on the paper or the like. When the oil coloring paint is stored in a container provided with the coating film-shaped multifunctional agent of the present invention, excessively rapid hardening of the liquid caused by oxidation of the drying oil can be prevented. Thus, a target oil coloring paint can be store for a long period of time, while a liquid condition ensuring use of the paint is maintained. Meanwhile, one type of coating materials is an "oxidation polymerization-type" coating material, which is dried through oxidation of the coating by reaction with oxygen in air. When a powder-form or granular-form multifunctional agent is dispersed in such an "oxidation polymerization-type" coating material, drying of the coating material may be retarded. In order to prevent oxidation of the "oxidation polymerization-type" coating material, a powder-form or granular-form multifunctional agent is not dispersed in the coating material but is allowed to stand in the coating material. Examples of preferred oxidation prevention method include storage of a coating material in a container having an inner wall on which the coating film-shaped multifunctional agent is provided and storage of a coating material in a container to which a bag including the multifunctional agent is added.

Also, the coating film-shaped multifunctional agent is not dissolved in an organic coating and does not modify the components of the organic coating. Therefore, even when the organic coating comes into contact with the multifunctional agent, the subtle color tone and the like of the organic coating are not affected, since the components of the organic coating are stable. Thus, the formed coating film containing a sufficient amount of the multifunctional agent satisfactorily suppresses deterioration of the organic coating, to thereby realize longer-term storage of organic coating without deterioration of the quality of the coating.

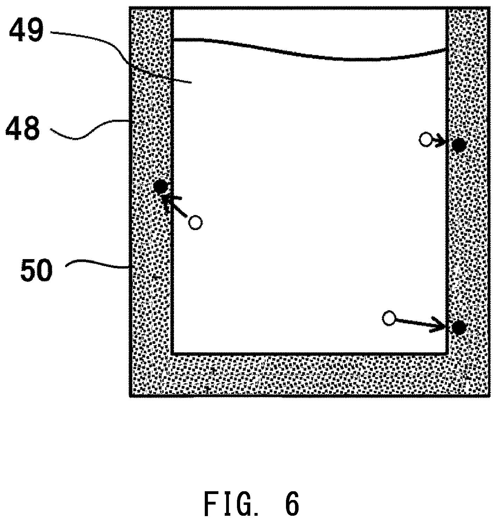

A specific embodiment of the multifunctional agent is a container-shaped multifunctional agent 48 as shown in FIG. 6. As described above, the container-shaped multifunctional agent 48 may be produced by preparing a slurry of a powder-form mayenite compound with an optional solvent, binder, etc., molding the slurry, and firing the molded product. As shown in FIG. 6, at least a part of an oil 49 contained in a container 50 comes into contact with the multifunctional agent 48 forming the container 50. In FIG. 6, a white circle denotes a substance present in the oil, such as oxygen ions, oxygen radicals, or oxidized hydrocarbon, while a black circle denotes a substance trapped by inclusion cages of the mayenite compound, such as oxygen ions or reduced hydrocarbon. Oxygen ions present in the oil are trapped by inclusion cages of the mayenite compound contained in the multifunctional agent 48 forming the container 50, whereby the oxidizing performance is lost. Also, oxygen radicals present in the oil are scavenged by transferring thereto electrons of the mayenite compound present in the multifunctional agent 48. Oxidized hydrocarbon present in the oil is reduced by electron or H⁻ enclosed in the mayenite compound. Thus, by storing a target oil or the like in the container 50 containing the multifunctional agent 48, oxidation of hydrocarbon in the oil can be prevented, whereby deterioration of the oil can be suppressed.

The container-shaped multifunctional agent is suitably used for storing a liquid whose oxidation and water inclusion are desirably prevented. Similar to the coating film-shaped multifunctional agent, the container-shaped multifunctional agent is suitably used for storing, for example, an organic coating such as ink or paint.

Then, an embodiment of the multifunctional agent used as, particularly, a reducing agent for oil will be described.

When the reducing agent is brought into contact with oil, an oxidized-form hydrocarbon, in which oxidation has proceeded, is reduced, to thereby regenerate a reduced-form hydrocarbon. By use of the reducing agent, a deteriorated waste oil in which oxidation of hydrocarbon has proceeded can be converted to a refined oil in which oxidation of hydrocarbon has not sufficiently proceeded. The reducing agent can reduce a hydrocarbon, when the agent donates electrons enclosed in the mayenite compound to the oxidized hydrocarbon present in oil. In addition, as a result of oxidation of hydrocarbon, a carbonyl compound or a similar compound is formed. The reducing agent feeds H⁻ enclosed in the mayenite compound to the carbonyl compound or the like, to thereby induce hydride reduction. As a result, the carbonyl compound or the like can be reduced to a corresponding alcohol.

An example of the aforementioned waste oil includes repeatedly used food oil and machine oils repeatedly used in factories. When such a waste oil is brought into contact with the multifunctional agent for a specific period of time, electron and H⁻ are donated to oxidized-form hydrocarbon species contained in the waste oil, to thereby produce refined reduced-from hydrocarbon. After regeneration of the reduced-form hydrocarbon, impurities contained in the waste oil are optionally removed for purification. Thus, refined oil which can be used again is yielded.

In many factories, repeatedly used machine oil is generally stored in a large-scale waste oil tank. To the waste oil tank, the ball-form or granular-form multifunctional agent itself or its product of the multifunctional agent incorporated into a mesh bag is fed, and the state is continued for a specific period of time. Alternatively, in order to enhance the efficiency of reaction between the waste oil and the multifunctional agent, the waste oil placed in the waste oil tank may be stirred. In the waste oil tank, an oxidized hydrocarbon is reduced by the action of the mayenite compound, to thereby form a reduced-form hydrocarbon. As a result, a refined oil which can be used again is yielded.

Hitherto, a large amount of used waste oil discharged from factories and the like is generally transferred to a large scale waste oil regeneration plant by means of an oil lorry. In such a waste oil regeneration plant, hydrogen gas is fed to the waste oil by means of a large blower, for carrying out reduction of hydrocarbon. Therefore, in conventional waste oil regeneration reaction, large facilities such as a waste oil regeneration plant and a blower are required. Thus, difficulty is encountered in readily regenerating waste oil in actual settings such as factories and homes, which is problematic. In addition, collecting waste oil and accumulation thereof require cumbersome work and high cost, which is also problematic.

By use of the multifunctional agent as a reducing agent, works and cost of collecting waste oil and accumulation thereof in factories can be reduced. Furthermore, in an actual factory which provides waste oil, waste oil can be regenerated by simply adding a ball-form or granular-form reducing agent to the tank.

Also, the reducing agent is suitably employed with respect to a domestic waste oil, such as a waste oil from a fry cooking tool such as a fryer. In a fryer for producing fried foods such as *tempura,* the used oil undergoes oxidation of hydrocarbon due to repeated cooking operations, and the oil used therein is unfavorably colored or generates unpleasant odor. Such a deteriorated waste oil is generally replaced with a new food oil product. Also, the waste oil is discharged as an industrial waste.

The waste oil from a fryer is stocked in an appropriate container, and the granular-form or ball-form multifunctional agent of the present invention is added to and allowed to stand in the container. Oxidized hydrocarbon present in the waste oil is reduced by the mayenite compound contained in the granular-form multifunctional agent. Through addition of a sufficient amount of the multifunctional agent and passage of a sufficient period of time, a large amount of oxidized hydrocarbon is reduced, whereby a recyclable refined oil can be obtained.

The multifunctional agent may be used as a reducing agent for domestic waste oil, whereby the amount of industrial waste including domestic waste oil can be reduced. As described above, the waste oil may be regenerated by simply allowing the granular-form multifunctional agent to stand in the waste oil. Thus, by use of the multifunctional agent, waste oil can be readily regenerated without any particular means (e.g., apparatus and operation). In addition, since the multifunctional agent of the present invention is not dissolved in waste oil, contamination of the regenerated oil with impurities can be prevented.

A specific embodiment of the multifunctional agent of the present invention is a filter-shaped multifunctional agent 28 as shown in FIG. 4. The filter-shaped multifunctional agent can be produced by molding a mixture of the powder-form mayenite compound with an optional solvent, binder, etc. into a shape of a target filter member. For example, the filter-shaped multifunctional agent may be a porous body having a shape of thin sheet, sheet, film, or column. Alternatively, the filter-shaped multifunctional agent may be formed by depositing the powder-form multifunctional agent on a nonwoven or woven fabric substrate. Yet alternatively, the filter-shaped multifunctional agent may be formed by filling a specific space with the powder-form or granular-form multifunctional agent.

As shown in FIG. 4, the filter-shaped multifunctional agent 28 is provided on, for example, a part or the entirety of the cross-section of a waste oil regeneration tube 30. From waste oil 29A on the upstream side of the filter-shaped multifunctional agent 28, regenerated oil 29B is obtained on the downstream side of the multifunctional agent. In order to sufficiently perform reduction reaction at the filter-shaped multifunctional agent 28, the thickness, porosity, waste oil 29A passing rate, etc. of the filter-shaped multifunctional agent 28 may be appropriately controlled, so that the regenerated oil 29B can be in contact with the filter-shaped multifunctional agent 28 for a sufficient time.

The filter-shaped multifunctional agent is suitably used in an oil filter automobile oil, machine oil, etc. The oil filter serves as a filter member for removing sludge, dust, etc. from a machine oil such as an automobile engine oil. In a more specific manner, the filter-shaped multifunctional agent of the invention may be incorporated into a part of a filter member of the oil filter. When an engine oil or a similar oil is caused to pass through the oil filter containing the filter-shaped multifunctional agent, dust and other undesired solid are removed. In addition, electron and H⁻ enclosed in the mayenite compound are transferred to the oil, thereby inducing reduction reaction of hydrocarbons contained in the oil. Furthermore, the mayenite compound reacts with water contained in the engine oil via hydration, to thereby realize dehydration of the oil. Thus, a regenerated oil can be obtained, by means of the oil filter, through reduction and dehydration of an engine oil deteriorated by oxidation of hydrocarbon. In the regenerated oil, oxidation of hydrocarbon and deterioration by water are suppressed to a considerable extent. By use of the filter-shaped multifunctional agent of the present invention, frequency of engine oil replacement can be reduced, to thereby reduce cost relating to engine oil replacement. Also, the generation of industrial waste originating from waste engine oil can be reduced.

The filter-shaped multifunctional agent may be used as a multifunctional agent which mainly acts as a reducing agent for regenerating waste oil from food oil used in a cooking apparatus such as a fryer. Specifically, the waste oil in which hydrocarbon components are oxidized is caused to pass through an oil filter containing the multifunctional agent. The oxidized hydrocarbon components contained in the waste oil are reduced during passage through the oil filter, and water in the waste oil is removed. By virtue of the oil filter, hydrocarbon is reduced, and water is removed. As a result, there is obtained a regenerated oil which can be reused as a food oil and whose deterioration is retarded. By use of the filter-shaped multifunctional agent of the present invention, the amount of waste oil from a cooking apparatus can be reduced, and frequency of replacement of food oil can be reduced, to thereby reduce cost relating to replacement of waste food oil. Also, the generation of industrial waste originating from waste food oil can be reduced.

The filter-shaped multifunctional agent of the present invention is not dissolved in oil when being in contact with the oil. Thus, the regenerated oil obtained through contact a waste machine oil with the multifunctional agent of the present invention contains only the components of the oil before regeneration and no impurity. As a result, the regenerated oil obtained by use of the multifunctional agent can serve as a machine oil, without causing any trouble in a target machine or the like.

In addition to modifying the quality of a target oil, the filter-shaped multifunctional agent has a function of modifying the quality of liquid or gas by passing the liquid or gas therethrough. For example, the filter-shaped multifunctional agent may be used as a filter having a radical scavenging activity for scavenging radicals present in air, a hazardous substance decomposition filter which can reduce hazardous (toxic) substances present in air for detoxification, or a CO₂ adsorption filter. In other words, the multifunctional agent may be suitably used as a gas phase-modifying agent. When air is caused to pass through the filter-shaped multifunctional agent, electrons enclosed in the inclusion cages of the mayenite compound are transferred to radicals present in air before passage through the filter, whereby the radical content of air can be reduced after passage through the filter. Also, when air is caused to pass through the filter-shaped multifunctional agent, electron and H⁻ enclosed in the inclusion cages of the mayenite compound are transferred to hazardous substances present in air before passage through the filter, to thereby reduce and detoxify the hazardous substances. Also, when air is caused to pass through the filter-shaped multifunctional agent, CO₂ present in air before passage through the filter is reduced, to thereby lower the CO₂ concentration of air, after passage through the filter.

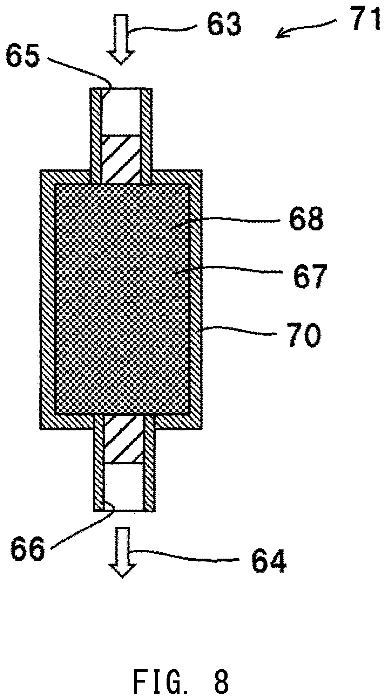

A specific embodiment of the multifunctional agent is a reactor-shaped multifunctional agent as shown in FIG. 8. In one mode of using the reactor-shaped multifunctional agent, waste oil can be regenerated. A reactor 71 of this embodiment has a casing 70 defined by two openings 65, 66 and an inner space 67, and includes a multifunctional agent 68 placed in the inner space 67. The embodiment of the multifunctional agent 68 having a granular form, a powder form, a ball form, a pellet form, or the like is not particularly limited to that charged in the inner space 67 as shown in FIG. 8, so long as it can be placed in the inner space 67. The reactor 71 may be realized an embodiment in which a coating film of the multifunctional agent is provided onto the inner wall of the reactor, an embodiment which has a porous body having a multifunctional agent of a shape similar to that of the inner space 67, or another embodiment. No particular limitation is imposed on the structure of the reactor 71, so long as the waste oil 63 can be passed through the reactor so as to be in contact with the multifunctional agent. Three or more openings may be provided. The shape of the casing 70 is not limited to the structure shown in FIG. 8, and a pipe extending along the axis direction, a hollow sphere, etc. may be employed. The waste oil 63 passed through the reactor 71 comes into contact with the multifunctional agent 68, whereby oxygen radicals in the waste oil 63 can be scavenged. Also, oxidized hydrocarbon present in the waste oil 63 is reduced by electron and H⁻ enclosed by the mayenite compound, to thereby refresh the waste oil 63. Water in the waste oil 63 reacts with the mayenite compound, to thereby form a hydrate, whereby water in the oil can be removed. In the case in which the multifunctional agent is a porous body, and the inner space 67 is filled with the multifunctional agent so as to complete charging a predetermined space, impurities in the waste oil 63 are trapped by passage through of the reactor 71. In this way, by passing the waste oil 63 through the reactor 71, a regenerated oil 64 is yielded. In the regenerated oil 64, oxidation of hydrocarbon and deterioration due to water are suppressed to a considerable extent. No particular limitation is imposed on the target oil, and examples include edible oil, engine oil, and insulating oil.

In addition to modifying the quality of a target oil to provide a regenerated oil, the reactor-shaped multifunctional agent has a function of modifying the quality of liquid (other than oil) or gas. The reactor 71 may be further provided with a mechanism of heating or pressurizing the waste oil 63 and the multifunctional agent 68. When the reactor 71 has a heating or pressurizing mechanism, reactivity regarding, for example, reduction reaction can be enhanced. The reactor 71 may be further provided with a mechanism of stimulating electrons enclosed in the mayenite compound contained in the multifunctional agent 68 (e.g., a mechanism of providing voltage, electromagnetic induction, electromagnetic wave, or the like). When the reactor 71 has a mechanism of providing voltage, electromagnetic induction, electromagnetic wave, or the like, to the mayenite compound, electric current flows through the multifunctional agent 68 placed in the casing 70, thereby enhancing reactivity. The multifunctional agent 68 contained in the reactor 71 may be further provided with a catalyst. When the catalyst is deposited on the multifunctional agent 68, the intrinsic activity of the mayenite compound is enhanced, thereby enhancing reactivity to reduction or the like. The reactor 71 may be further provided with a filter, an adsorption layer, or the like for capturing microparticles on the downstream side of the multifunctional agent. When the reactor 71 has a filter, an adsorption layer, or the like for capturing microparticles, a hydrate formed through hydration reaction of the mayenite compound contained in the multifunctional agent with water is captured, whereby incorporation of impurities into the regenerated oil 64 passed through the reactor 71 can be prevented.

During use of the multifunctional agent of the present invention for a long period of time, as the aforementioned antioxidant, reducing agent, or the like, electron or H⁻ enclosed in the mayenite compound is substituted by ions other than H⁻. As a result, the electron content or the H⁻ content of the mayenite compound gradually decreases. When the enclosed electron content or the H- content of the mayenite compound has decreased, the electron donation performance of the multifunctional agent is lowered. In other words, during use of the multifunctional agent, the antioxidation performance, reduction performance, etc. of the multifunctional agent are gradually reduced. Meanwhile, the mayenite compound is hydrated via contact with water. Thus, even when active species enclosed by inclusion cages in the surface portion of the cage cluster have decreased, enclosed active species enclosed in the inside of the cage cluster induce reduction reaction or the like upon breakage of the crystal cage through hydration reaction. Therefore, in the case where the multifunctional agent is used in the presence of water, the antioxidation performance, reduction performance, etc. of the multifunctional agent can be attained for a long period of time. The rate of reaction between active species enclosed in the mayenite compound and other radicals, ions, etc. increases in response to an increase in amount of water in contact with the mayenite compound, since the amounts of active species released from crystal cages increase by breakage of the crystal cages. Thus, when the multifunctional agent is used in an aqueous medium, reaction rate is high. In contrast, when the multifunctional agent is used under the conditions where the water content of oil or the like is small, reaction rate is moderate. However, due to deterioration of oil, the mayenite compound is hydrated by the formed water, to thereby release active species enclosed in cage clusters. As a result, the functions of the multifunctional agent can be attained for a long period of time. Also, when the multifunctional agent is used for modifying the quality of a gas phase, the mayenite compound is hydrated by vapor contained in combustion gas, exhaled air, etc., to thereby release active species enclosed in cage clusters. As a result, the functions of the multifunctional agent can be attained for a long period of time.

As described in the above modes and embodiments, the multifunctional agent is used in a hydrophobic solvent such as oil. However, needless to say, the multifunctional agent of the present invention may be used in a hydrophilic solvent such as water. The multifunctional agent is not dissolved in water and exhibits oxidation prevention performance, reduction performance, radical scavenging activity, etc. Specifically, the multifunctional agent can reduce various organic substances present in water, to thereby scavenge radicals therein.

Next, a particular case where the multifunctional agent is used as an agent having radical scavenging activity will be described.

The multifunctional agent serving as an agent having radical scavenging activity contains the mayenite compound I enclosing both electron and H⁻, and/or the mixture II of a mayenite compound enclosing electron and a mayenite compound enclosing and H⁻. Among them, the mayenite compound I and the mixture II, each having high electron donating performance; i.e., having a high enclosed electron density, are suitably employed. In preservation or storage of chemicals containing an organic compound such as a pharmaceutical product, an agricultural agent, a reagent, or the like, active oxygen may generate from oxygen molecules present in air, thereby possibly inducing radical reaction. Through such a radical reaction, an organic compound contained in the above chemical is decomposed, to reduce the amount of an ingredient of the chemical. As a result, functions, effects, and the like of chemical may be impaired. Conventionally, in order to prevent decomposition or deterioration of such an organic compound, the container therefore is sealed so as to prevent entry of oxygen into the container. However, when oxygen molecules unavoidably enter the container, the oxygen molecules serve as starting points of radical reaction, to thereby possibly cause decomposition of organic compounds.

The multifunctional agent may be used as an agent having radical scavenging activity for preventing radical reaction occurring in storage of a target drug. In a specific manner, the granular-form or ball-form multifunctional agent of the invention is added to the container accommodating the drug, whereby the radical reaction can be prevented. For example, in storage of a solid drug, a bag including the granular-form multifunctional agent or the ball-form multifunctional agent is added to the container accommodating the target drug. When electrons enclosed in the mayenite compound contained in the multifunctional agent are transferred to oxygen radicals present in gas, the oxygen radicals are transformed into oxygen ions, whereby the oxygen radicals are scavenged. Also, a portion of the oxygen ions are trapped by inclusion cages, whereby oxygen ions are enclosed. Thus, in the case where the multifunctional agent scavenges oxygen radicals among present radicals, the agent may also be called an active oxygen scavenger. When the mayenite compound contained in the multifunctional agent is in contact with water, a hydrate is formed via hydration reaction, to thereby attain dehydration performance. Therefore, the multifunctional agent can remove water contained in air, whereby deterioration of a target drug, which would otherwise be caused by water contained in air, can be prevented.

In order to store a liquid-form drug, a bag including the granular-form multifunctional agent or the ball-form multifunctional agent may be added to the liquid-form drug. In this case, when electrons enclosed in the mayenite compound contained in the multifunctional agent are transferred to oxygen radicals present in liquid, to thereby transform the oxygen radicals into oxygen ions, whereby the oxygen radicals are scavenged. Also, a portion of the oxygen ions are trapped by inclusion cages, whereby oxygen ions can be enclosed.

As described above, the multifunctional agent serving as an agent having radical scavenging activity can prevent radical reaction in a drug which might be decomposed and deactivated by the radical reaction, whereby the drug can be stored for a long period of time, without deactivating the drug. In addition, since the multifunctional agent is insoluble in any liquid, the agent can serve as an agent having radical scavenging activity, without lowering the purity of the target drug, even when the agent is added to the drug in liquid form.

Meanwhile, a coating film-shaped multifunctional agent as shown in FIG. 5 or a container-shaped multifunctional agent as shown in FIG. 6 may be used as the agent having radical scavenging activity. In a specific mode, when a drug is stored in a drug storage container having an inner wall formed of the coating film-shaped multifunctional agent or in a drug storage container formed of a material containing the multifunctional agent, radical reaction in the container leading to deactivation of the drug can be prevented. The multifunctional agent also exhibits dehydration performance, by virtue of formation of a hydrate via hydration through contact of the mayenite compound of the multifunctional agent with water. Thus, the multifunctional agent can remove water from air or a liquid-form drug, whereby deterioration of the drug, which would otherwise be caused by water contained in the drug, can be prevented.

Next, an embodiment of the multifunctional agent for use as a tobacco smoke filter will be described.

More specifically, the aforementioned filter-shaped multifunctional agent may be incorporated into a part of a tobacco filter, to thereby provide a tobacco smoke filter. In one embodiment, the multifunctional agent is placed as a part of a filter in the vicinity of tobacco leaves. The multifunctional agent may be in the form of a columnar or thin-sheet porous body, or a powder or granules charged into a frame. During smoking, various radical species such as active oxygen generate. By use of the filter-shaped multifunctional agent, the radical content of tobacco smoke can be reduced. In addition, hazardous substances such as a carcinogen contained in tobacco smoke can be detoxified. Also, the CO₂ concentration of tobacco smoke can be reduced. Meanwhile, water vapor present in combustion gas generated by tobacco reacts with the crystal cages of the mayenite compound, causing hydration. As a result, crystal cages present in a surface portion of the cage cluster are broken, thereby releasing active species enclosed in the crystal cages present inside the cage cluster. Thus, the aforementioned reaction continues. Therefore, by use of the filter-shaped multifunctional agent of the present invention, the levels of hazardous substances contained in smoke, such as radicals, carcinogens, and CO₂, can be reduced.

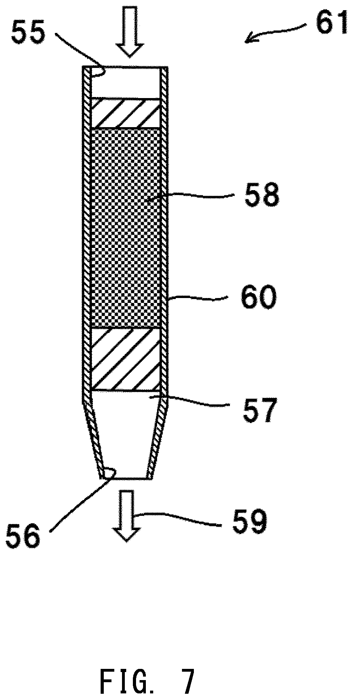

In the case where the multifunctional agent is used for modifying the quality of tobacco smoke, the use of the multifunctional agent is not limited to a part of a tobacco filter, and the agent may be used as, for example, a part of a pipe-shaped attachment for tobacco smoke. As shown in an embodiment of FIG. 7, an attachment for tobacco smoke 61 has a casing 60 provided with an inner space 57 and two openings 55, 56, and includes a multifunctional agent 58 placed in the inner space 57. In use of the attachment for tobacco smoke, a cigarette is attached to the opening 55, and a smoker smokes it through the other opening 56. When tobacco smoke 59 passing through the casing comes into contact with the multifunctional agent 58, the quality of the smoke is modified. The multifunctional agent 58 placed in the inner space 47 may be a porous body or a powder or granules thereof charged into a frame. In the case where the multifunctional agent is used as a part of a tobacco smoke filter or an attachment for tobacco smoke, the multifunctional agent in the filter or attachment is preferably connected to a microparticle-capturing filter, a microparticle-adsorbing layer, or the like, on the downstream side of the tobacco smoke. The reason for this is as follows. Water vapor present in combustion gas generated by tobacco reacts with the mayenite compound, and microparticles of the formed hydrate may possibly generate. When a microparticle-capturing filter or a microparticle-adsorbing layer that can capture microparticles of hydrates is placed in the vicinity of the multifunctional agent, intake of the hydrate microparticles by the human body can be prevented.

Next, an embodiment of the multifunctional agent in a filter part of a mask or a gas mask will be described.

The filter-shaped or the reactor-shaped multifunctional agent may also be used in a filter member of a conventional mask or gas mask. A typical example of the mask has a fabric filter member having such an area that can cover the mouth and the nose, and strings for donning the mask. The fabric filter member containing the multifunctional agent may be formed by depositing the multifunctional agent onto a non-woven fabric substrate, which is the same manner as employed in dying nonwoven fabric or the like. A gas mask typically has a mask body for covering the face of a user, a belt for fixing the mask body to the head of the user, and a reactor for modifying the quality of a hazardous gas via passage of the gas through the reactor. An example of the structure of the reactor is shown in FIG. 8. That is, the reactor is has a casing provided with two openings and an inner space. In the inner space, the porous body of the multifunctional agent is placed, or a powder or granules of the multifunctional agent are charged in the space. When the hazardous gas passes through the multifunctional agent placed in the reactor, the quality of the hazardous gas is modified. When the hazardous gas passes through a mask or gas mask employing the filter-shaped multifunctional agent, radicals contained in the hazardous gas are scavenged; hazardous substances contained in the hazardous gas are detoxified; and CO₂ is removed. Therefore, by use of a mask or gas mask employing the filter-shaped or reactor-shaped multifunctional agent, the mask user does not inhale air containing large amounts of radicals, hazardous substances, and/or CO₂. Also, the multifunctional agent is hydrated by water; i.e., water vapor contained in exhaled air or water contained in air, whereby crystal cages in a surface portion of the cage cluster and inside the cage cluster are broken. As a result, enclosed active species present inside the crystal cages can be involved in reaction, to thereby maintain the performance of modifying the quality of hazardous gas for a long period of time. Notably, the filter-shaped or reactor-shaped multifunctional agent adsorbs CO₂ but releases CO at 200°C or higher. Thus, the multifunctional agent is preferably used at a temperature lower than 200°C.

Next, an embodiment of the multifunctional agent for use as an organic compound decomposing agent will be described.

The multifunctional agent serving as an organic compound decomposing agent contains a mayenite compound enclosing at least H⁻. That is, the multifunctional agent contains at least one of the mayenite compound I enclosing both electron and H⁻, the mixture II, and a mayenite compound enclosing H⁻, and such a mayenite compound preferably has high H⁻ donating performance.

The organic compound decomposing agent transfers H⁻ enclosed in the mayenite compound to a target organic compound, to thereby induce hydride reduction, leading to decomposition of the organic compound. In one specific mode, the organic compound decomposing agent can decompose a volatile organic compound such as formaldehyde, which is a substance possibly causing "sick building syndrome." Meanwhile, the volatile organic compound (e.g., formaldehyde) room level is restricted by various regulations. However, those who are sensitive to such a chemical substance may have a bad reaction to formaldehyde at a level lower than a regulated value. Thus, there are some commercial housing products which physically adsorb formaldehyde, the products containing zeolite, a porous material, gel, beads, charcoal, and the like. Since these products reduce the formaldehyde level through physical adsorption, adsorption performance reaches a plateau in the case where pores and the like are in an adsorption saturated state. Thus, consistent adsorption effect may fail to be attained in some cases. In contrast, the organic compound decomposing agent of the invention releases H⁻ enclosed in the mayenite compound and transfers H⁻ to a volatile organic compound (e.g., formaldehyde), thereby inducing hydride reduction. As a result, the target organic compound is reduced to an alcohol, whereby the volatile organic compound present in the room can be detoxified. Meanwhile, no particular limitation is imposed on the volatile organic compound to be decomposed, so long as the compound undergoes hydride reduction. Examples of the compound include carbonyl compounds having a carbonyl group such as formaldehyde, acetaldehyde, and acetone.

No particular limitation is imposed on the form of the organic compound decomposing agent, and examples include building materials such as sheet, wallpaper, coating in slurry form, and board, coating agent, granule, powder, ball, filter, porous body, and an apparatus having an air-circulation mechanism employing a reactor filled with the mayenite compound of the invention. The aforementioned organic compound decomposing agent may be formed singly of a mayenite compound, or a mixture of the mayenite compound with other optional materials. In order to enhance reactivity, a catalyst may be added to the mayenite compound. The apparatus employing a reactor filled with the mayenite compound of the invention or a similar apparatus may further include a heating mechanism or a pressurizing mechanism for enhancing reactivity.

Next, an embodiment of the multifunctional agent for use as a hydrogen supplying agent will be described.

The multifunctional agent serving as a hydrogen supplying agent contains a mayenite compound enclosing at least H⁻. That is, the multifunctional agent contains at least one of the mayenite compound I enclosing both electron and H⁻, the mixture II, and a mayenite compound enclosing H⁻, and such a mayenite compound preferably has high H- donating performance.

The mayenite compound is dissolved in acid. When a mayenite compound enclosing H⁻ is dissolved in acid, hydrogen gas is generated. Also, hydration of the mayenite compound via contact with water results in breakage of crystalline cages, to thereby generate hydrogen gas. When a tablet of the mayenite compound and an optional substance is perorally administered to a human subject, H⁻ enclosed in the mayenite compound is released by gastric acid and water of the subject. When hydrogen is present as an aqueous solution or in a certain medium, hydrogen readily becomes volatile. However, hydrogen enclosed in the mayenite compound in the form of tablet or the like can be reliably maintained, to thereby provide a hydrogen supplement having excellent portability. Meanwhile, a hydrate formed through hydration of the mayenite compound is a cement component, which is not digested and absorbed by the human body. Thus, even when the mayenite compound is perorally administered as a hydrogen supplying agent, it is discharged with feces from the body. That is, the hydrogen supplying agent is non-toxic to the human body. Also, since the mayenite compound enclosing H⁻ releases hydrogen gas through hydration, the above hydrogen supplying agent may be added to various beverages such as water, tea, and refreshing beverages, to thereby provide drinkable hydrogen water. In yet another use, the hydrogen supplying agent may be added as a bath salt, to thereby provide a hydrogen-containing bath.

No particular limitation is imposed on the form of the hydrogen supplying agent, and examples thereof include powder, granule, pellet, filter, container, porous body, and a bag containing the hydrogen supplying agent. The above hydrogen supplying agents may be formed singly of a mayenite compound or a mixture of the mayenite compound with other optional materials.

Next, an embodiment of the multifunctional agent for use as a hydrogen occlusion body will be described.

The multifunctional agent serving as a hydrogen occlusion body contains a mayenite compound enclosing at least H⁻. That is, the multifunctional agent contains at least one of the mayenite compound I enclosing both electron and H⁻, the mixture II, and a mayenite compound enclosing H⁻, and such a mayenite compound preferably has high H⁻ donating performance.

Due to a small molecular size, hydrogen gas must be stored in a specially designed container for reliable storage. From another aspect, the H⁻-enclosing mayenite compound stores hydrogen in the form of hydrogen anion. Thus, when the mayenite compound is dissolved in acid, hydrated, or heated at 200°C to 550°C, hydrogen gas can be generated. Since the mayenite compound is solid at ambient temperature, the hydrogen occlusion body is effective in terms of hydrogen portability and storage capacity. Meanwhile, hydrogen gas occluding alloys, which conventionally have been developed for the purpose of hydrogen storage, are heavy and disadvantageous for portability and other purposes. In contrast, the mayenite compound is formed of light elements, and its weight is advantageously small. Furthermore, the mayenite compound is also available as powder or granule, thereby attaining easy handling. Since the mayenite compound can generate hydrogen under specific conditions, the compound may find uses including a hydrogen tank of a hydrogen automobile, a hydrogen storage module employed in hydrogen stations, hydrogen fuel storage, and a portable hydrogen source for use in a small-scale fuel cell or the like.

Next, an embodiment of the multifunctional agent for use as a recording medium will be described.

The multifunctional agent serving as a recording medium contains a mayenite compound enclosing H⁻, and the recording medium is formed by molding the H⁻-enclosing mayenite compound to a shape of interest, preferably a sheet. When the surface of the recording medium is irradiated with a UV ray, an X-ray, an electron beam, or the like, the mayenite compound of the thus-irradiated portion is converted to an electron-enclosing mayenite compound. By virtue of this property, a molded product of the H⁻-enclosing mayenite compound can serve as a recording medium. In the recording medium formed of the H⁻-enclosing mayenite compound, the mayenite compound of the irradiated (UV or the like) portion is converted to an electron-enclosing mayenite compound. Based on a continuously or discretely arranging pattern of a plurality of the thus-provided portions, information can be recorded (written). Examples of the means for writing information on the recording medium include a UV laser, an X-ray laser, and an electron beam, and examples of the mode of application thereof onto the recording medium include continuous irradiation and dot-mode irradiation. The aforementioned lasers or electron beam realizes a very fine writing feature. Thus, the recording medium realizes mass information storage. Also, mass information storage can also be attained by the recording medium, since each chemical species enclosed in one mayenite compound molecule can be employed as an information unit. Meanwhile, among electron-enclosing mayenite compounds, C12A7 releases electrons at a temperature higher than 300°C, and is returned to an H⁻-enclosing mayenite compound. Therefore, the information written into the recording medium is removed by heating the medium at a temperature higher than 300°C, and then new information can be stored in the recoding medium. In addition, the H⁻-enclosing mayenite compound is converted to the corresponding electron-enclosing mayenite compound through irradiation with a UV ray or the like. Thus, the recording medium preferably has a protective member which can intercept a UV ray or the like, similar to that case of the below-mentioned electronic circuit substrate. The recording medium is free from a UV ray or the like, except for irradiation with a UV ray or the like for the purpose of information recording. So long as the recording medium is used at 300°C or lower, the written information can be stored in a semi-permanent manner.

Next, an embodiment of the multifunctional agent for use as a soil spray agent will be described.

The multifunctional agent serving as a soil spray agent contains a mayenite compound enclosing at least one of electron and H⁻. That is, the multifunctional agent contains at least one of the mayenite compound I enclosing both electron and H⁻, the mixture II, a mayenite compound enclosing electron, and a mayenite compound enclosing H⁻.

The soil spray agent of the invention can detoxify a hazardous substance or a malodorous substance via reduction by transferring electron and/or H⁻ enclosed in the mayenite compound to the target substance present in soil. The electron-enclosing mayenite compound reduces hydrocarbon by transferring electrons thereto, whereas the H⁻-enclosing mayenite compound transfers H⁻ to a carbonyl compound or the like, to thereby induce hydride reduction. As a result, the carbonyl compound or the like is reduced to a corresponding alcohol. Thus, when the electron-enclosing mayenite compound and the H⁻-enclosing mayenite compound are selectively employed in accordance with the type of hazardous substance and malodorous substance, with the balance of the two compounds being appropriately tuned, the effect of the soil spray agent can be further attained. In the case where water is present in soil, electrons enclosed in the mayenite compound are transferred to water, to thereby generate OH⁻. As a result, the pH of the soil increases. Therefore, the electron-enclosing mayenite compound can modulate the pH of the target soil. The soil spray agent may also be advantageously used as a pH-adjusting agent for the purpose of plant growth, which is controlled by the pH of the soil. When the mayenite compound reacts with water, a hydrate (a cement component) is formed. Therefore, if the mayenite compound is continuously present in soil, no problem of toxicity occurs. No particular limitation is imposed on the form of the soil spray agent, and examples thereof include powder, powder-dispersed solution, granule, ball, and pellet.

Next, an embodiment of the multifunctional agent for use as a deodorant will be described.

The multifunctional agent serving as a deodorant contains a mayenite compound enclosing at least one of electron and H⁻. That is, the multifunctional agent contains at least one of the mayenite compound I enclosing both electron and H⁻, the mixture II, a mayenite compound enclosing electron, and a mayenite compound enclosing H⁻.

The deodorant can remove malodor by transferring electron and/or H⁻ enclosed in the mayenite compound to a target odorous substance, to thereby reduce the substance. The target malodorous substance may be in the form of solid, liquid, or gas. The electron-enclosing mayenite compound can reduce hydrocarbon by transferring electrons thereto. The H⁻-enclosing mayenite compound can transfer H⁻ to a carbonyl compound or the like, to thereby induce hydride reduction. As a result, the carbonyl compound or the like is reduced to a corresponding alcohol species. Thus, when the electron-enclosing mayenite compound and the H⁻-enclosing mayenite compound are selectively employed in accordance with the type of malodorous substance, with the balance of the two compounds being appropriately tuned, the effect of the deodorant can be further attained. No particular limitation is imposed on the form of the deodorant, and examples thereof include powder, granule, ball, pellet, and filter. The deodorant may be used as a shoe inner sole, a diaper, a sanitary napkin, a sweat-absorbing pad, or cloth, a coating or spray coating liquid for animals and humans, and a toilet deodorant.

Next, an embodiment of the multifunctional agent for use as a dehydrating agent/deoxygenating agent will be described.

The multifunctional agent serving as a dehydrating agent/deoxygenating agent contains a mayenite compound enclosing at least electron. That is, the multifunctional agent contains at least one of the mayenite compound I enclosing both electron and H⁻, the mixture II, and a mayenite compound enclosing electron, and such a mayenite compound preferably has high electron donating performance.

As already known, dehydrating agents and deoxygenating agents may be used for removing oxygen and water contained in food, drugs, machine-operating atmosphere, and liquid. Hitherto, dehydrating agents such as silica gels and quick lime have been used for hydration of target substances, and deoxygenating agents have been used for only absorbing oxygen in targets. Thus, such a dehydrating agent or a deoxygenating agent has a single function. In contrast, at least the electron-enclosing mayenite compound is hydrated to form a hydrate, whereby water can be removed from a target. As a result, the above agent can serve as a dehydrating agent and a deoxygenating agent, which can transfer electrons to oxygen radicals present around the mayenite compound, and can take oxygen ions. In other words, the mayenite compound containing at least electron can have a function of a dehydrating agent and a deoxygenating agent. No particular limitation is imposed on the form of the dehydrating/deoxygenating agent, and examples thereof include bag containing powder or granule, ball, container, and inner coating for a container cap. The dehydrating/deoxygenating agent is preferably employed in the use where a deoxygenating agent is used, and further dehydration performance is needed. Examples of the case include suppression of growth of aerobic bacteria and molds, prevention of food oxidation, and deterioration of cloth.

Next, an embodiment of the multifunctional agent for use as a corrosion inhibitor will be described.

The multifunctional agent serving as a corrosion inhibitor contains a mayenite compound enclosing at least electron. That is, the multifunctional agent contains at least one of the mayenite compound I enclosing both electron and H⁻, the mixture II, and a mayenite compound enclosing electron, and such a mayenite compound preferably has high electron donating performance.

Generally, in order to prevent corrosion of metal, contact between metal with oxygen and water must be inhibited. In this regard, the electron-enclosing mayenite compound transfers electrons enclosed therein to radicals (in particular, oxygen radicals) present outside the cage cluster, whereby highly reactive radicals, in particular, active oxygen, can be scavenged. As a result, oxygen ions are trapped, to thereby prevent oxidation reaction. The mayenite compound exhibits dehydration performance via hydration through contact with water. Thus, when a coating material containing a corrosion inhibitor is applied onto a metallic target object, corrosion can be retarded by removing oxygen and water. No particular limitation is imposed on the form of the corrosion inhibitor, and examples thereof include powder, granule, bag including powder or granule, sheet in which powder is dispersed, and coating containing powder.

Next, an embodiment of the multifunctional agent for use as a fire retardant will be described.

The multifunctional agent serving as a fire retardant contains a mayenite compound enclosing at least electron. That is, the multifunctional agent contains at least one of the mayenite compound I enclosing both electron and H⁻, the mixture II, and a mayenite compound enclosing electron, and such a mayenite compound preferably has high electron donating performance.

Combustion is defined as a reaction in which a generated radical further reacts with oxygen in air, to thereby further increase radicals in a chain radical reaction manner. The electron-enclosing mayenite compound transfers electrons to radicals present outside the cage cluster, to thereby scavenge highly reactive radicals. As a result, chain radical reaction can prevented. Alternatively, through incorporation of oxygen into the mayenite compound, the oxygen concentration can be lowered. Also, when the mayenite compound is hydrated with water vapor contained in combustion gas or the like, crystal cages present in a surface portion of the cage cluster are broken, whereby enclosed electrons inside the crystal cages can be involved in reaction. No particular limitation is imposed on the fire retardant, and examples thereof include powder, granule, and sheet in which powder is dispersed. Needless to say, the fire retardant of the invention may be used in combination with other fire retardants.

Next, an embodiment of the multifunctional agent for use as an electromagnetic wave-absorbing material will be described.

The multifunctional agent serving as an electromagnetic wave-absorbing material contains a mayenite compound enclosing at least electron. That is, the multifunctional agent contains at least one of the mayenite compound I enclosing both electron and H⁻, the mixture II, and a mayenite compound enclosing electron, and such a mayenite compound preferably has high electron donating performance. Also, the multifunctional agent is more preferably a black body of the mayenite compound having high enclosed electron concentration.

In the electron-enclosing mayenite compound, enclosed electrons freely move between crystal cages similar to the case of metal elements. Thus, the electron-enclosing mayenite compound has the same properties as those of metals. Particularly, the electron-enclosing mayenite compound absorbs electromagnetic waves having various wavelengths and can serve as an electromagnetic wave-absorbing material. Furthermore, the electron-enclosing mayenite compound may be used as an electromagnetic wave-shielding material, by virtue of its property of absorbing electromagnetic waves. No particular limitation is imposed on the electromagnetic wave-absorbing material, and examples thereof include coating material, coating agent, powder, container, glass, and ceramic. The electromagnetic wave-absorbing material may be used as a building material such as brick, wall panel, window pane, and roof tile.

Next, an embodiment of the multifunctional agent for use as an electron emission source will be described.

The multifunctional agent serving as an electron emission source contains a mayenite compound enclosing at least electron. That is, the multifunctional agent contains at least one of the mayenite compound I enclosing both electron and H⁻, the mixture II, and a mayenite compound enclosing electron, and such a mayenite compound preferably has high electron donating performance. Also, the multifunctional agent is more preferably a black body of the mayenite compound having high enclosed electron concentration.

The electron-enclosing mayenite compound can emit electrons upon application of an electric field, thereby serving as an electron emission source of an electron beam generator. Such an electron beam generator may be employed as a power source. Particularly when the electron beam generator is employed as a chargeable power source of an astronautic apparatus, no thrust agent is needed. In addition, since the mayenite compound is formed of light elements and has a small mass, the weight of the target astronautic apparatus can be reduced. Alternatively, the electron-enclosing mayenite compound may be employed as an electron emission source for use in a neutralization apparatus of an ion engine.

Next, an embodiment of the multifunctional agent for use as a filler for column chromatography will be described.

The multifunctional agent serving as a filler for column chromatography contains a mayenite compound enclosing at least electron. That is, the multifunctional agent contains at least one of the mayenite compound I enclosing both electron and H⁻, the mixture II, and a mayenite compound enclosing electron, and such a mayenite compound preferably has high electron donating performance. Also, the multifunctional agent is more preferably a black body of the mayenite compound having high enclosed electron concentration.

The technique of chromatography includes gas chromatography, liquid chromatography, and supercritical fluid chromatography. In ion-exchange chromatography, which is one liquid chromatography technique, ionic compounds can be separated from one another based on the difference of the amount of charge of the compounds. Since the electron-enclosing mayenite compound emits electron via hydration, a negatively charged species is generated. Such an electron-enclosing mayenite compound may be used as an stationary phase of a column of ion-exchange chromatography. Also, the mayenite compound enclosing at least one of electron and H⁻ incorporates ions such as oxygen ion into crystal cages and undergoes hydration with water. That is, the compound exhibits dehydration performance. Therefore, when such a mayenite compound is used at a specific site in a chromatographic system, possible background components (e.g., water and oxygen) can be removed. Thus, the mayenite compound can serve as a dehydrating agent and a deoxygenating agent in chromatography, leading to correct analysis. In addition, in the presence of water, the electron-enclosing mayenite compound transfers enclosed electrons to water, to thereby generate OH⁻, whereby the pH of the mobile phase can be controlled. A specific embodiment of the column has an stationary phase produced by filling a cylindrical member with a mayenite compound in the form of powder or granule. If needed, the stationary phase may further include silica gel, active alumina, etc. The active species enclosed by the mayenite compound is not limited to electron and H , and the below-mentioned oxygen ions and the like may also be employed in accordance with the purpose of use.

Next, an embodiment of the multifunctional agent in a thermoelectric device will be described.

The multifunctional agent used in a thermoelectric device contains a mayenite compound enclosing at least electron. That is, the multifunctional agent contains at least one of the mayenite compound I enclosing both electron and H⁻, the mixture II, and a mayenite compound enclosing electron, and such a mayenite compound preferably has high electron donating performance. Also, the multifunctional agent is more preferably a black body of the mayenite compound having high enclosed electron concentration.

In the electron-enclosing mayenite compound, enclosed electrons freely move between crystal cages similar to the case of metal elements. Thus, the electron-enclosing mayenite compound has the same properties as those of metals. Thus, the electron-enclosing mayenite compound may be employed as a Peltier device, a Thompson device, or a Seebeck device. An embodiment of the Peltier device is realized by joining a piece of a first mayenite compound to a piece of a second mayenite compound having an electron concentration different from that of the first mayenite compound, to thereby form a joined body. Through passage of electric current, an exothermal or endothermal process can be induced. In an embodiment of the Thompson device, when an electric current is caused to flow through a wire material of a mayenite compound having a specific electron concentration and a temperature gradient, an exothermal or endothermal process in proportion to the current can be induced. Thus, the Peltier device and the Thompson device can be employed in a heating apparatus, a cooling apparatus, and the like. An embodiment of the Seebeck device is realized by joining a piece of a first mayenite compound to a piece of a second mayenite compound having an electron concentration different from that of the first mayenite compound, to thereby form a circularly joined body (i.e., a closed circuit). When the two joining portions are maintained at different temperatures, electromotive force can be provided between the two joining portions. In other words, the Seebeck device can transform thermal energy to electric energy, and thus serves as a thermoelectric generator.

Next, an embodiment of the multifunctional agent for use as a catalyst will be described.

The multifunctional agent used as a catalyst preferably contains a mayenite compound enclosing at least electron. That is, the multifunctional agent preferably contains at least one of the mayenite compound I enclosing both electron and H⁻, the mixture II, and a mayenite compound enclosing electron, and such a mayenite compound more preferably has high electron donating performance. Also, the multifunctional agent is particularly preferably a black body of the mayenite compound having high enclosed electron concentration.

By virtue of the electron donation property, the multifunctional agent can be used as a reduction catalyst. In one specific embodiment of the catalyst, the multifunctional agent is used as a reduction catalyst for use in a CO₂ decomposition filter that can reduce carbon dioxide to carbon monoxide. In another embodiment, the multifunctional agent is used as a material of an ammonia synthesis catalyst for producing ammonia from N₂ and H₂.

Next, an embodiment of the multifunctional agent for use as a glass for producing optical fiber (hereinafter may be referred to as an "optical fiber glass") will be described.

The multifunctional agent used as an optical fiber glass contains any mayenite compound, preferably a mayenite compound enclosing at least electron. That is, the multifunctional agent contains a mayenite compound containing any active species, preferably at least one of the mayenite compound I enclosing both electron and H⁻, the mixture II, and a mayenite compound enclosing electron.

Through melting and quenching, the mayenite compound can be vitrified. Thus, glass products and articles having a variety of shapes can be formed from a mayenite compound. Being drawn, the mayenite compound provides an optical fiber and can be used as either a core or a clad of the optical fiber. By virtue of its electric conductivity, the electron-enclosing mayenite compound can provide a cable which can transmit optical signals and electric signals and feed electric power.

Next, an embodiment of the multifunctional agent for use as a brazing filler material will be described.

The multifunctional agent used as a brazing filler material contains any mayenite compound, preferably a mayenite compound enclosing at least electron. That is, the multifunctional agent contains a mayenite compound containing any active species, preferably at least one of the mayenite compound I enclosing both electron and H⁻, the mixture II, and a mayenite compound enclosing electron, and such a mayenite compound preferably has high electron donating performance. Also, the multifunctional agent is particularly preferably a black body of the mayenite compound having high enclosed electron concentration.

Through melting and quenching, the mayenite compound can be vitrified. Thus, the mayenite compound can serve as a glass adhesive for use in welding such as soldering or brazing. Conventionally, when ceramic substrates are bonded with a metallic adhesive which has conventionally been employed in welding, the two substrates may possibly be defoliated from each other by change in temperature, due to the difference in thermal expansion coefficient between metal and ceramic material. In contrast, since the mayenite compound is a ceramic material, when the mayenite compound is used as a glass adhesive for bonding ceramic substrates, the difference in thermal expansion coefficient between metal and ceramic material can be reduced. As a result, peeling due to change in temperature can be prevented. In addition, by virtue of electric conductivity, the electron-enclosing mayenite compound can be used as a glass adhesive having electric conductivity.

Next will be described an embodiment of the H⁻-enclosing mayenite compound as a composition for producing an electronic circuit substrate, and an embodiment of the H⁻-enclosing mayenite compound and the electron-enclosing mayenite compound for producing an electronic circuit substrate.

The composition for producing an electronic circuit substrate contains an H⁻-enclosing mayenite compound. Firstly, the electronic circuit substrate producing composition is molded to form a substrate, and the substrate is irradiated with a UV ray, an X-ray, or an electron beam. As a result, the H⁻-enclosing mayenite compound in the irradiated portion to a corresponding electron-enclosing mayenite compound. Thus, the irradiated portion is endowed with electric conductivity. Through converting the H⁻-enclosing mayenite compound in a target portion of the substrate is converted to a corresponding electron-enclosing mayenite compound, an electronic circuit substrate having a circuit of a desired pattern is yielded. That is, the thus-yielded electronic circuit substrate includes a substrate containing the H⁻-enclosing mayenite compound, and an electronic circuit which is formed on the substrate and which contains the electron-enclosing mayenite compound. No particular limitation is imposed on the method for forming the circuit, and examples of the method include irradiation of a substrate whose specific area is masked with a UV ray or the like, and irradiation of a non-surface-masked substrate with a UV ray laser, an X-ray laser, or an electron beam, in a continuous or dot-like manner. Among them, formation of a circuit by means of a laser or an electron beam is preferred, since a fine circuit pattern can be formed. No particular limitation is imposed on the form of the mayenite compound forming the substrate, and examples thereof include single crystal, polycrystal, glass, and ceramic.

Being oxidized by oxygen present in air, the mayenite compound enclosing electron or H⁻ tends to exhibit an increase in electric resistance. Therefore, the electronic circuit substrate formed from the mayenite compound enclosing H⁻ and electron preferably has a protective member that can prevent oxidation. No particular limitation is imposed on the form of the protective member, and examples thereof include a protective coating film which covers an electronic circuit substrate so as to intercept entry of oxygen, and a protective case which covers the electronic circuit substrate so as to intercept entry of oxygen. Examples of the protective coating film include a metallic protective coating film (i.e., metal coating film for protection use). The metallic coating film may be formed on the surface of an electronic circuit substrate through metal vapor deposition, metal spraying, or a similar technique. Preferably, the container of the protective case is filled with inert gas, so long that the container can seal the circuit-provided surface.

The electronic circuit substrate formed from an H⁻-enclosing mayenite compound and the electron-enclosing mayenite compound absorbs a UV ray or the like upon exposure to light. As a result, the H⁻-enclosing mayenite compound forming the substrate containing is converted to a corresponding electron-enclosing mayenite compound, whereby the electronic circuit may be deactivated. Thus, the electronic circuit substrate formed from the H⁻-enclosing mayenite compound and the electron-enclosing mayenite compound preferably has a protective member that can intercept an electromagnetic wave having a specific wavelength (e.g., UV rays or X-ray). No particular limitation is imposed on the form of the protective member, and examples thereof include a protective coating film that can cover the electronic circuit substrate so as to intercept an electromagnetic wave of a specific wavelength, and a protective case that can cover the electronic circuit substrate so as to intercept an electromagnetic wave of a specific wavelength. The protective coating film may be a metallic coating film; i.e., a metal coating film. For example, such a metal film may be formed on the surface of the electronic circuit substrate through a technique such as metal vapor deposition or metal spraying. The protective case may be a container having a cap on which at least a circuit is provided. The protective case may be formed of a material that reflects an electromagnetic wave having a certain wavelength.

Among the aforementioned electron-enclosing mayenite compounds, C12A7 releases enclosed electrons at a temperature higher than 300°C, failing to maintain its electric conductivity. Thus, when an electronic circuit substrate formed from the H⁻-enclosing mayenite compound and the electron-enclosing mayenite compound is operated at a temperature higher than 300°C, a protective member is preferably provided so as not to exceed the temperature of the electronic circuit substrate of 300°C. Examples of the protective member include an adiabatic material that can intercept heat and a cooling apparatus that can cool the electronic circuit substrate. No particular limitation is imposed on the adiabatic material, and examples thereof include a protective coating film covering the electronic circuit substrate, which coating film intercepts heat. Examples of the cooling apparatus include a cooling fan which realizes heat dissipation through blowing.

Next will be described an embodiment of the H⁻-enclosing mayenite compound and the electron-enclosing mayenite compound for use as a fuse.

In the electron-enclosing mayenite compound, enclosed electrons freely move between crystal cages similar to the case of metal elements. Thus, the electron-enclosing mayenite compound has the same properties as those of metals, thereby realizing electric conductivity. Among electron-enclosing mayenite compounds, C12A7 releases enclosed electrons at a temperature higher than 300°C, thereby converting to an insulator. Based on this property, an electronic circuit substrate formed from the H⁻-enclosing mayenite compound and the electron-enclosing mayenite compound may be used as an electric fuse. If a target electronic circuit is designed so that the circuit is heated to a temperature higher than 300°C upon flow of large current, the circuit becomes an insulator upon flow of surge current, to thereby break the circuit. Thus, the circuit of the invention can serve as an electric fuse. Examples of the means for controlling the temperature of the electronic circuit to higher than 300°C include modifying the electron concentration of the mayenite compound forming the circuit, and controlling the wire diameter of circuit. Through any of these means, the electric resistance of the circuit can be appropriately tuned.

Conventionally, a fuse has a metal wire enclosed in a glass tube, and such a fuse is readily damaged by vibration or impact. In contrast, the fuse formed from a mayenite compound is a circuit containing an electron-enclosing mayenite compound formed on a substrate containing an H⁻-enclosing mayenite compound. Thus, the fuse of the embodiment of the invention has higher resistance to vibration and impact, as compared with conventional fuses. Also conventionally, a fuse is provided on a substrate as a separated part. However, as mentioned above, the fused formed from the mayenite compound can be fabricated through irradiating a substrate containing an H⁻-enclosing mayenite compound with a UV ray or the like, to thereby form an electronic circuit. Thus, the fuse can be formed monolithically with the electronic circuit substrate.

Next an embodiment of the H⁻-enclosing mayenite compound and the electron-enclosing mayenite compound for use in a magnetic field generator will be described.

As described above, an electronic circuit is formed, through irradiation with a UV ray or the like, on a substrate made of an H⁻-enclosing mayenite compound, to thereby provide an electronic circuit substrate. Further, through passage of a current through an electronic circuit having a pattern of interest (e.g., coil-like or linear), a magnetic field can be generated. Thus, the electronic circuit substrate having an appropriate electronic circuit pattern may serve as a magnetic field generator. No particular limitation is imposed on the form of the magnetic field generator, and the form is not limited to substrate. Other examples include electronic circuits having an appropriate pattern made of a coating film on a substrate having a shape of column, tube, or the like. The magnetic field generator may be employed in an electromagnet, a motor, a power generator, a speaker, etc. Since the mayenite compound is formed on elements lighter than metallic elements, a lightweight magnetic field generator can be provided.

Next, an embodiment of the H⁻-enclosing mayenite compound and the electron-enclosing mayenite compound for use in a heater employing an enclosed mayenite compound will be described.

Through irradiation of an H⁻-enclosing mayenite compound with a UV ray, an X-ray, an electron beam, or the like, the irradiated portion is converted to a corresponding electron-enclosing mayenite compound. Thus, the electron-enclosing mayenite compound has electrical conductivity and the same properties as those of metals. Through irradiating the H⁻-enclosing mayenite compound with a UV ray or the like, a circuit formed of an electron-enclosing mayenite compound can be yielded. By passing electricity through the circuit serving as a resistor, heat can be generated. Examples of the means for forming a resistor having a resistance of interest from the electron-enclosing mayenite compound include appropriately controlling the electron concentration of the mayenite compound, and appropriately adjusting the width of each trace of the circuit. Meanwhile, conventional heaters are produced by use of a heating wire. Typically, a heater of a heating wire is formed on a ceramic substrate. Due to difference in thermal expansion coefficient between the substrate and the electric wire, the heater may be cracked. In contrast, the substrate, and the resistor and resistor wire of the heater can be produced from the mayenite compound of the invention, and cracking, which would have been caused by the difference in thermal expansion coefficient, can be prevented. The thus-produced heater may be employed as a defogger of glass articles, an air heater, a water heater, an oil heater, a heater for a heating device, and the like.

Next, an embodiment of the H⁻-enclosing mayenite compound and the electron-enclosing mayenite compound for use in an antenna will be described.

The electron-enclosing mayenite compound has an electric conductivity and the same properties as those of metal. Thus, through designing a circuit of an antenna structure adapted to an electromagnetic wave having a wavelength of interest (e.g., radio wave, microwave, infrared ray, or visible light), the produced antenna can radiate (to a space) or receive an electromagnetic wave. By irradiating the H⁻-enclosing mayenite compound with a laser light (e.g., UV) or an electron beam, a fine circuit is formed. The circuit can serve as an antenna. The antenna formed from a mayenite compound can radiate or receive an electromagnetic wave (e.g., IR ray or visible light) other than radio wave. No particular limitation is imposed on the form of the mayenite compound for providing an antenna, and examples of the form include single crystal, polycrystal, glass, coating film, and ceramic. No particular limitation is imposed on the shape of the antenna, and examples thereof include wire, sheet, plate, and coil.

The antenna formed from a mayenite compound may be employed in an electromagnetic wave transmitter and an electromagnetic wave receiver.

Through designing the antenna circuit so as to radiate an IR ray, the antenna formed from a mayenite compound may serve as an IR-radiating antenna. The antenna may be employed in an IR-transmitter. The IR-transmitter may also be employed as a heating device.

Through designing the antenna circuit so as to radiate visible light, the antenna formed from a mayenite compound may serve as a visible-light-radiating antenna. The antenna may be employed in a visible-light-transmitter.

The antenna formed from a mayenite compound may be employed as a light source which can directly emit visible light having any wavelength, differing from the case of a fluorescent light or the like, which converts a UV ray generated upon electric discharge to visible light through contact with a phosphor body.

Through designing the antenna circuit so as to radiate an electromagnetic wave having a single frequency, the antenna formed from a mayenite compound may serve as a laser-transmitting element, which may be employed in a laser-transmitter.

Through designing the antenna circuit so as to radiate visible light, the antenna formed from a mayenite compound may serve as a light source that can emit visible light. The light source may be employed as a light source for use in a display device.

Through designing the antenna circuit so as to receive an electromagnetic wave having a specific wavelength, the antenna formed from a mayenite compound may serve as an antenna that can receive an electromagnetic wave having a specific wavelength. When the electromagnetic wave received by the electromagnetic wave receiver is transformed into an electric signal, an image sensor for use in a camera can be provided.

The electromagnetic wave which has been received by the antenna can be transformed into electric power. Thus, through transforming the electromagnetic wave having any wavelength which has been received by the antenna formed from a mayenite compound into electric power, a new-type electric generator can be provided. The antenna formed from a mayenite compound can receive visible light or an IR ray, which has a longer wavelength, and can convert the light to electric power. Thus, through placing the antenna in the vicinity of any of various heat sources, excessive heat can be transformed into electric power.

Next, an embodiment of the H⁻-enclosing mayenite compound and the electron-enclosing mayenite compound for use in a temperature sensor will be described.

In the electron-enclosing mayenite compound, enclosed electrons freely move between crystal cages similar to the case of metal elements. Thus, the electron-enclosing mayenite compound has the same properties as those of metals, and the electric resistance thereof increases with increase in temperature. Based on this property, a temperature sensor can be provided by combination of an electronic circuit substrate formed from an H⁻-enclosing mayenite compound and an electron-enclosing mayenite compound with a resistance meter for measuring electric resistance. Among electron-enclosing mayenite compounds, C12A7 releases electrons at a temperature higher than 300°C, thereby releasing enclosed electrons. In this case, the properties intrinsic to metal fail to be maintained. Thus, the temperature sensor can be operated at 300°C or lower.

In the case where the electron donating activity decreases, the electron donating activity of the multifunctional agent can be revived through a regeneration treatment. In one specific procedure, the multifunctional agent having a reduced electron donating activity is placed in, for example, a noble metal crucible under a reducing atmosphere (e.g., hydrogen gas), and the noble metal crucible is heated, whereby the anion species other than H⁻ enclosed in the mayenite compound can be converted to H⁻. Alternatively, the multifunctional agent having a reduced electron donating activity is placed in a carbon crucible, and the carbon crucible is heated, whereby the anion species enclosed in the mayenite compound can be converted to electrons. The heating temperature in the noble metal crucible or the carbon crucible may be appropriately modified in accordance with the type of the anion species enclosed in the mayenite compound. In one mode, the heating temperature is 700°C to 1,450°C. Furthermore, when the multifunctional agent containing H⁻-substituted a mayenite compound is irradiated with a UV ray or the like, H⁻ is partially substituted by electrons. As described above, the multifunctional agent having a reduced electron donating activity can be transformed into a multifunctional agent enclosing a large amount of H⁻ and electron having revived electron donating activity. The thus-obtained insoluble mayenite compound exhibiting revived electron donating activity may be reused as an antioxidant, a reducing agent, a deoxygenating agent, a radical scavenging activator, etc.

In the case of the aforementioned granular-form or ball-form multifunctional agent, used granules or balls of the agent, exhibiting reduced electron donating activity, are placed in, for example, a noble metal crucible in a reducing atmosphere (e.g., hydrogen gas), to thereby induce substitution to H⁻. Then, the agent is irradiated with a UV ray or the like, whereby the electron donating activity of the granular-form or ball-form multifunctional agent is revived. In the case of the filter-shaped multifunctional agent, used filters containing the agent, exhibiting reduced electron donating activity, are placed in, for example, a noble metal crucible in a reducing atmosphere (e.g., hydrogen gas), to thereby induce substitution to H⁻. Then, the agent is irradiated with a UV ray or the like, whereby the electron donating activity of the filter-shaped multifunctional agent is revived. In the case of the coating film-shaped multifunctional agent, the coating film is separated from the inner wall of the container. The separated coating film member is placed in, for example, a noble metal crucible in a reducing atmosphere (e.g., hydrogen gas), to thereby induce substitution to H⁻. Then, the agent is irradiated with a UV ray or the like. To the thus-irradiated coating film member, a solvent, a binder, or the like is added to form a slurry, and the slurry is applied onto the inner wall of the container and fixed, whereby the electron donating activity of the filter-shaped multifunctional agent is revived.

The multifunctional agent according to the present invention can be repeatedly used through a regeneration process, differing from conventional one-way-use antioxidants. By use of the multifunctional agent according to the present invention, the amount of the waste originating from the antioxidant can be reduced. Notably, the hydrated mayenite compound cannot be regenerated only through the aforementioned reduction treatment.

As described above, particularly when the mayenite compound contains Ca, Al, and O in the crystal lattice, the color tone of the mayenite compound contained in the multifunctional agent of the present invention varies in accordance with the enclosed electron content. Specifically, as the enclosed electron content increases, the mayenite compound becomes black, whereas as the enclosed electron content decreases, the mayenite compound becomes pale black. Also, when crystal cages of the mayenite compound are broken through hydration, the color of the compound becomes pale black. Thus, whether or not the multifunctional agent of the present invention has an electron donating activity can be clearly determined through observing the color tone of the agent. Specifically, immediately after use of the multifunctional agent, the mayenite compound thereof encloses a large amount of electrons and assumes a thick black color. In the course of use of the multifunctional agent for a long period of time, electrons enclosed in the mayenite compound gradually decrease, whereby the black color of the multifunctional agent is gradually weakened. After passage of a certain period of time, the black multifunctional agent is completely decolored. That is, the compound assumes virtually white. Through checking of white color of the multifunctional agent, the multifunctional agent can be determined to fail to maintain electron donating activity. The thus-assessed multifunctional agent may be replaced by a new multifunctional agent, or can be regenerated through, for example, the aforementioned regeneration treatment so as to attain enclosing electron and H⁻ again in the mayenite compound, whereby the electron donating activity can be revived. Therefore, the timing of replacement or regeneration of the multifunctional agent of the present invention is immediately and clearly determined through observation of the color tone thereof.

The mayenite compound contained in the multifunctional agent may be synthesized from inexpensive raw powder materials of CaO and Al₂O₃. Thus, as compared with conventional antioxidants, reducing agents, deoxygenating agents, agents having radical scavenging activity, etc., a multifunctional agent can be mass-produced from the multifunctional agent of the present invention containing the mayenite compound at lower cost. Notably, the multifunctional agent of the present invention can be produced from a commercial C12A7 powder.

### Mayenite compound enclosing active oxygen species

The mayenite compound enclosing active oxygen species such as O⁻ and O₂⁻ causes oxidation by transferring active oxygen species to molecules and ions other than the mayenite compound. Thus, the mayenite compound enclosing active oxygen species may be used as an oxidizing agent.

The mayenite compound enclosing active oxygen species has a cage-like crystal structure in which negatively charged active oxygen species are enclosed in positively charged inclusion cages. The inclusion cages can remove water through hydration with water and incorporate anions thereinto.

When the inclusion cages are broken through hydration, the mayenite compound enclosing active oxygen species releases enclosed active oxygen species. As the hydration proceeds, not only crystal cages at the surface of the cage cluster but also those inside the cage cluster are broken. As a result, enclosed active oxygen species inside the crystal cages can also be involved in the reaction. Thus, when the mayenite compound enclosing active oxygen species is used in the presence of water, the activity of the oxidizing agent or the like can be maintained for a long period of time.

Since the mayenite compound enclosing active oxygen species is a ceramic inorganic compound, the compound is present in a solid state in liquid and is not dissolved in an organic solvent, particularly in oil.

Except that the particles of active oxygen species are enclosed in inclusion cages, the mayenite compound enclosing active oxygen species has the same structure as that of the mayenite compound I enclosing electron and H⁻. Examples of the active oxygen species include O⁻ and O₂⁻. In inclusion cages, the active oxygen species are present in various oxidation states, such as O²⁻, O⁻, O₂²⁻, and O₂⁻. Similar to the mayenite compound I, the mayenite compound enclosing active oxygen species may include anions and radicals other than active oxygen species as unavoidable impurities.

Whether the mayenite compound encloses active oxygen species may be confirmed by the presence of a signal attributed to O⁻ and O₂⁻ in an ESR spectrum measured by means of an electron spin resonance (ESR) spectrometer.

### Form of mayenite compound enclosing active oxygen species

The mayenite compound enclosing active oxygen species may be in any form. Examples of the form include container, porous body, coating film, powder, granular, ball, pellet, and filter. The form of the active oxygen species-enclosing mayenite compound is the same as described in relation to the aforementioned "The form of the mayenite compound I and the mixture II."

### Method of producing mayenite compound enclosing active oxygen species

The mayenite compound enclosing active oxygen species may be produced through the same method as described in the aforementioned part "production of mayenite compound I enclosing electron and H⁻ and mixture II." The same procedure of heating a powder-form or granular-form mayenite compound precursor itself or after a molding step, in a reducing atmosphere, to thereby produce the H⁻-enclosing mayenite compound, may be repeated, except that dry oxygen gas is caused to flow at 1,200°C to 1,400°C. Upon heating, as the partial oxygen pressure increases, or the partial vapor pressure decreases, a mayenite compound having a high active oxygen species concentration can be yielded.

### Use of mayenite compound enclosing active oxygen species

The mayenite compound enclosing active oxygen species (e.g., O⁻ and O₂⁻) has an oxidizing activity. Thus, the mayenite compound enclosing active oxygen species may be used as an oxidizing agent. Actually, the mayenite compound enclosing active oxygen species can oxidize an organic compound in the form of solid, liquid, or gas. Through oxidation of such an organic compound, a hazardous substance and a malodorous substance can be oxidized for detoxification.

Next, an embodiment of the mayenite compound enclosing active oxygen species for use as an organic compound decomposing agent will be described.

The organic compound decomposing agent can oxidize an organic compound by transferring active oxygen enclosed in the mayenite compound to the organic compound. In one specific mode, the organic compound decomposing agent can oxidize a volatile organic compound such as formaldehyde, which is a substance possibly causing "sick building syndrome." The organic compound decomposing agent can oxidize formaldehyde to form formic acid, which is then oxidized and decomposed to form water and carbon dioxide, to thereby attain detoxification. In the case where the atmosphere contains water vapor, the mayenite compound enclosing active oxygen species is hydrated. As a result, not only crystal cages at the surface of the cage cluster but also those inside the cage cluster are broken. Thus, enclosed active oxygen species inside the crystal cages can also be involved in the reaction. Thus, when the multifunctional agent is used in the presence of water, the function of detoxifying a hazardous substance and a malodorous substance can be maintained for a long period of time.

No particular limitation is imposed on the form of the organic compound decomposing agent. Examples thereof include sheet, wall paper, slurry form coating, building material such as board, coating agent, granule, powder, ball, filter, porous body, and an apparatus having an air circulator employing a reactor as shown in FIG. 8. These organic compound decomposing agents of the above forms may be formed of a mayenite compound enclosing the active oxygen species or from a mixture thereof with an optional material. In addition, in order to enhance reactivity, a catalyst may be deposited on the mayenite compound enclosing active oxygen species. Alternatively, an apparatus such as a reactor filled with the mayenite compound enclosing the active oxygen species may be further provided with a heating mechanism or a pressurizing mechanism, for enhancing reactivity.

Next, an embodiment of the mayenite compound enclosing active oxygen species for use as a soil spray agent will be described.

The soil spray agent of the invention can detoxify a hazardous substance or a malodorous substance via oxidation by transferring active oxygen species enclosed in the mayenite compound to the target substance present in soil. In the case where water is present in soil, the mayenite compound enclosing active oxygen species is hydrated. As a result, not only crystal cages at the surface of the cage cluster but also those inside the cage cluster are broken. Thus, enclosed active oxygen species inside the crystal cages can also be involved in the reaction, and the function of detoxifying a hazardous substance and a malodorous substance can be maintained for a long period of time. When the active oxygen species-enclosing mayenite compound reacts with water, a hydrate (a cement component) is formed. Therefore, if the mayenite compound is continuously present in soil, no problem of toxicity occurs. No particular limitation is imposed on the form of the soil spray agent, and examples thereof include powder, powder-dispersed solution, granule, pellet, and ball.

Next, an embodiment of the active oxygen species-enclosing mayenite compound for use as an antibacterial agent will be described.

The active oxygen species-enclosing mayenite compound can sterilize bacteria and viruses. No particular limitation is imposed on the antibacterial agent, and examples thereof include powder, powder-dispersed solution, granule, pellet, ball, filter, sheet, and reactor. If the antibacterial agent is sprayed onto soil, pond, and other sites, where a bacterium with high pathogenicity or a virus providing high fatality is identified, expansion of infection by such a bacterium or virus can be prevented. The antibacterial agent may be formed into a filter or a reactor. When such an antibacterial agent is used in a filter member of a mask or gas mask, intake of bacteria, virus, and the like by the user can be prevented. The mayenite compound enclosing active oxygen species is hydrated with water vapor contained in air or exhaled air. As a result, not only crystal cages at the surface of the cage cluster but also those inside the cage cluster are broken. Thus, enclosed active oxygen species inside the crystal cages can also be involved in the reaction, whereby the function of removing bacteria, viruses, etc. can be maintained for a long period of time. The form and other properties of the mask or gas mask are the same as described above.

Next, an embodiment of the active oxygen species-enclosing mayenite compound for use as a gas phase-modifying agent will be described.

The gas phase-modifying agent can modify the quality of any gas phase via oxidation. More specifically, the gas phase-modifying agent can modify the target gas phase by detoxifying, through oxidation, a hazardous substance or a malodorous substance contained in air, exhaust gas of an automobile or the like, factory exhaust gas, tobacco smoke, etc. and removing bacteria, virus, etc. In the case where the gas phase contains water vapor, the mayenite compound enclosing active oxygen species is hydrated with water vapor contained in air or exhaled air. As a result, not only crystal cages at the surface of the cage cluster but also those inside the cage cluster are broken. Thus, enclosed active oxygen species inside the crystal cages can also be involved in the reaction, whereby the function of modifying the quality of the target gas phase can be maintained for a long period of time. No particular limitation is imposed on the form of the gas phase-modifying agent, and examples thereof include filter, reactor, powder, granule, ball, and pellet.

Next, an embodiment of the active oxygen species-enclosing mayenite compound for use as a deodorant will be described.

The deodorant can remove malodor by transferring active oxygen species enclosed in the mayenite compound to a target odorous substance, to thereby oxidize the substance. The target malodorous substance may be in the form of solid, liquid, or gas. Thus, when the electron-enclosing mayenite compound, the H⁻-enclosing mayenite compound, and the active oxygen species-enclosing mayenite compound are selectively employed in accordance with the type of malodorous substance, with the balance among the three compounds being appropriately tuned, the effect of the deodorant can be further attained. No particular limitation is imposed on the form of the deodorant, and examples thereof include powder, granule, ball, pellet, filter, and reactor. The deodorant may be used as a material of a shoe inner sole, a diaper, a sanitary napkin, a sweat-absorbing pad, or cloth, a coating or spray coating liquid for animals and humans, and a toilet deodorant.

Next, an embodiment of the active oxygen species-enclosing mayenite compound as an insulator for use in spark plugs and an inner wall material for internal combustion engines will be described.

Generally, a spark plug includes a rod-shaped center electrode and terminals which are arranged along the center axis, a substantially cylindrical insulator provided externally of the center electrode and terminals, a substantially cylindrical metallic shell provided externally of the insulator, and a ground electrode attached to an end of the metallic shell. A gap is provided between an end of the center electrode and and end the ground electrode to allow spark discharge to occur upon application of high voltage. The spark plug is set in a combustion chamber of an internal combustion engine such that the gap allowing spark discharge to occur is located in the chamber. A small gap is present between the insulator provided externally of the center electrode and the metallic shell, and, in some cases, unburnt fuel and carbon are deposited on the insulator. In the case where carbon or the like is deposited on the insulator, insulation resistance decreases, resulting in electrical passage at a site other than the gap. In this case, an accidental fire may occur.

At a temperature higher than about 700°C, the active oxygen species-enclosing mayenite compound releases active oxygen species from inclusion cages, thereby promoting oxidation. In operation, the temperature in the combustion chamber of an internal combustion engine reaches about 3,000°C in some cases. In the case where the insulator of the spark plug is formed of an active oxygen species-enclosing mayenite compound, unburnt fuel and carbon are deposited on the insulator and are oxidized by the active oxygen species released from inclusion cages, to thereby form water and carbon dioxide. Thus, an accidental fire, which would otherwise be caused by a drop in insulation resistance of the insulator, can be prevented. Also, in the case where the inner wall of the combustion chamber of an internal combustion engine is formed of an active oxygen species-enclosing mayenite compound, active oxygen species enclosed in the mayenite compound are released to the combustion chamber, as the temperature inside the chamber increases through operation thereof. The thus-released active oxygen species serve as free radicals. As a result, the free radical concentration increases during combustion of the fuel, thereby leading to enhanced combustion performance. In addition, carbon deposited on the inner wall of the combustion chamber is oxidized by active oxygen species released from inclusion cages, to thereby form carbon monoxide and carbon dioxide, whereby carbon can be removed from the inner wall of the combustion chamber.

Next, an embodiment of the active oxygen species-enclosing mayenite compound for use as an O⁻ beam emission source will be described.

Through application of an electric field to a mayenite compound having high active oxygen concentration, high-density O⁻ ion beam can be generated. Thus, the active oxygen species-enclosing mayenite compound may be employed as an O⁻ beam emission source of an O⁻ beam generator. Through continuously feeding oxygen gas to the active oxygen species-enclosing mayenite compound, an O⁻ beam can be continuously emitted from the active oxygen species-enclosing mayenite compound. Also, through applying an electric field to the active oxygen species-enclosing mayenite compound while it is heated at about 700°C or higher, a pure O⁻ beam can be obtained.

### EXAMPLES

### Production of oxygen-containing anion-enclosing mayenite compound precursor

Raw material powders of CaCO₃ and Al₂O₃ were mixed at a mole ratio of 12 : 7, and the powder mixture and ethanol were added to a resin pot. The resultant mixture agitated by means of a rotator, to thereby prepare a slurry. The slurry was heated in hot water, to thereby evaporate ethanol, whereby a powder mixture was yielded. Subsequently, the powder mixture was placed on a firing dish, and the dish was placed in an electric furnace. The mixture was fired in air by heating the mixture at a temperature elevation rate of 4°C/minute and maintaining the mixture at 1,300°C for 10 hours. The thus-fired product was pulverized in a mortar, and fine particles are recovered through sieving, to thereby yield a mayenite compound precursor A. Through X-ray diffractometry, the mayenite compound precursor A was identified as C12A7 having a cage-like crystal structure.

### Production of electron- and H⁻-enclosing mayenite compound I

The mayenite compound precursor A and ethanol were added to a beaker, and the contents were agitated by means of a rotator, to thereby prepare a slurry. The slurry was heated in hot water, to thereby evaporate ethanol, whereby a powder mixture was yielded. Subsequently, the powder mixture was placed in a carbon crucible, which was then placed in an electric furnace. Under a flow of hydrogen, the mixture was fired by heating the mixture at a temperature elevation rate of 4°C/minute and maintaining the mixture at 1,300°C for 24 hours, to thereby yield a sample B.

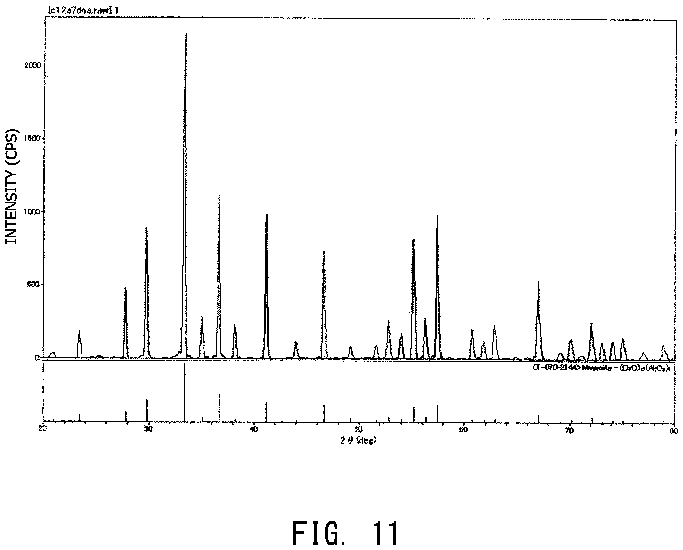

The sample B was analyzed by means of an X-ray diffractometer, and an X-ray diffraction pattern shown in the upper section of FIG. 11 was obtained. Through comparison of the obtained X-ray diffraction pattern with the X-ray diffraction pattern of the mayenite compound (C12A7) (ICDD card) shown in the lower section of FIG. 11, the sample B was identified as a mayenite compound (C12A7) having a cage-like crystal structure. Through visual observation, the sample B assumed green. In the case where the mayenite compound encloses no electron, the powder of the compound is white. As the enclosed electron concentration increases, the color of the powder becomes yellow, yellowish green, green, and black, in this order. Therefore, the sample B was found to enclose electrons at high concentration. The ESR spectrum of the sample B was measured by means of an electron spin resonance apparatus (ESR: product of BRUKER, Elexsys E580). In a specific measurement procedure, the sample B was placed in a quartz tube (inner diameter: 4 mm), and the atmosphere was changed to vacuum, and then He. The measurement was performed at room temperature. As a result, a signal conceivably attributed to electron (g = about 1.9944) was observed. Also from this information, the sample B was found to enclose electrons. When the sample B was placed in hydrochloric acid or water, gas generation was observed in both cases. Since the mayenite compound was obtained through firing in a reducing atmosphere, the gas was thought to be hydrogen. Thus, it is estimated at high possibility that the sample B encloses hydrogen ions. In conclusion, the sample B was found to be the mayenite compound I enclosing both electron and H⁻.

### Production of mixture II of electron-enclosing mayenite compound and H⁻-enclosing mayenite compound

The mayenite compound precursor A and ethanol were added to a beaker, and the contents were agitated by means of a rotator, to thereby prepare a slurry. The slurry was heated in hot water, to thereby evaporate ethanol, whereby a powder mixture was yielded. Subsequently, the powder mixture was placed in a carbon crucible, which was then placed in an electric furnace. Under a flow of nitrogen, the mixture was fired by heating the mixture at a temperature elevation rate of 4°C/minute and maintaining the mixture at 1,300°C for 24 hours, to thereby yield a sample C. Notably, a dehydrating agent was placed in a gas feeding route for feeding nitrogen to the electric furnace, and firing was performed in a dry atmosphere of the furnace.

Separately, a powder mixture prepared in a manner similar to that employed in production of sample C was placed in an iridium crucible, which was then placed in an electric furnace. Under a flow of hydrogen, the mixture was fired by heating the mixture at a temperature elevation rate of 4°C/minute and maintaining the mixture at 1,300°C for 24 hours, to thereby yield a sample D. Notably, a dehydrating agent was placed in a gas feeding route for feeding hydrogen to the electric furnace, and firing was performed in a dry atmosphere of the furnace.

The samples C and D were subjected to the same X-ray diffractometry as performed in the case of the sample B. The samples C and D were identified as a mayenite compound (C12A7).

Through visual observation, the sample C assumed green. Thus, the sample C was found to enclose electrons.

That is, the sample C is a mayenite compound enclosing electron.

When water was added to the sample D, gas was generated from the sample D. Since the mayenite compound was obtained through firing in a reducing atmosphere, the gas was thought to be hydrogen. Thus, it is estimated at high possibility that the sample D encloses hydrogen ions.

In conclusion, the sample D was found to be the mayenite compound enclosing H⁻.

Finally, the samples C and D and ethanol were added to a beaker, and the contents were agitated by means of a rotator, to thereby prepare a slurry. The slurry was heated in hot water, to thereby evaporate ethanol, whereby the mixture II of the electron-enclosing mayenite compound and the H⁻-enclosing mayenite compound was yielded.

### Assessment of reducing property

An edible oil was used as a test sample. The acid value of the edible oil sample was determined by use of an acid value test paper. The acid value was 2.0, indicating that the edible oil was oxidized.

The sample B was crushed in a mortar to form a powder. The powder-form sample B and edible oil were mixed together, to thereby prepare a mixture slurry.

Several minutes after preparation of the mixture slurry, the acid value of the edible oil in the mixture was measured, and the value was 1.0. The greater the acid value, the more severe the oil deterioration. Thus, the oxidized oil was found to be reduced by the sample B.

### Assessment of dehydrating property

The sample B was crushed in a mortar to form a powder, and water was added to the powder-form sample B. Several minutes after the start of this test, generation of fine bubbles was confirmed, and the particle size of the sample B was reduced. Thus, the sample B was hydrated through contact with water, to thereby provide fine hydrate particles. That is, through addition of the sample B to a water-containing oil or the like, the sample B is hydrated, to thereby remove water from the oil.

The mixture II was subjected to the same test. Several minutes after the start of this test, generation of fine bubbles was confirmed, and the particle size of the mixture II was reduced.

### Production of glass-form mayenite compound enclosing electron and H⁻

The sample B was crushed in a mortar to form a powder, and the powder-form sample B was placed in an alumina dish. The sample was then heated in an electric furnace at a temperature elevation rate of 4°C/minute under a flow of hydrogen gas. Then, the sample B was maintained at 1,600°C for 6 hours, to thereby melt the sample B, and the molten material was allowed to stand, to thereby yield a vitreous sample E.

The sample E assumed pale green, indicating that the sample E was found to enclose electrons. When hydrochloric acid was added to the sample E, hydrogen gas was generated from the sample E, indicating that the sample E was found to enclose hydrogen ions.

Thus, a glass-form mayenite compound enclosing electron and H⁻ was found to be formed by melting the sample B.

### DESCRIPTION OF REFERENCE NUMERALS

2 ··· O
3 ··· Ca
4 ··· Al
5 ··· electron
6 ··· inclusion cage
7 ··· bag
8 ··· granular-form multifunctional agent
9, 19, 39, 49 ··· oil
10, 20, 40, 50 ··· container
18 ··· ball-form multifunctional agent
28 ··· filter-shaped multifunctional agent
29A ··· waste oil
29B ··· regenerated oil
30 ··· waste oil regeneration tube
38 ··· coating film-shaped multifunctional agent
48 ··· container-shaped multifunctional agent
55, 56, 65, 66 ··· opening
57, 67 ··· inner space
58, 68 ··· multifunctional agent
59 ··· smoke
60, 70 ··· casing
61 ··· attachment for tobacco smoke
63 ··· waste oil
64 ··· regenerated oil
71 ··· reactor
72 ··· iron core
73 ··· coil
78, 88 ··· bag
79, 89 ··· insulating oil
80 ··· transformer tank
81 ··· oil-immersed transformer
82 ··· capacitor
90 ··· capacitor tank
91 ··· oil-impregnated capacitor

## Claims

1. A mayenite compound **characterized by** enclosing electron and H⁻.

2. A multifunctional agent, **characterized by** containing at least one of a mayenite compound I enclosing electron and H⁻, and a mixture II of a mayenite compound enclosing electron and a mayenite compound enclosing H , and exhibiting radical scavenging activity, reducing property, deoxygenation performance, and dehydration performance.

3. A container, **characterized by** containing, at least as a part thereof, a multifunctional agent as recited in claim 2.

4. A container according to claim 3, which is made of a ceramic or a glass.

5. A porous body, **characterized by** comprising a multifunctional agent as recited in claim 2, and having at least a porous surface.

6. A coating film, **characterized by** comprising a multi-functional agent as recited in claim 2.

7. A filter, **characterized by** comprising a multi-functional agent as recited in claim 2.

8. A reactor, **characterized by** comprising a casing at least having two openings and an inner space, and a multifunctional agent as recited in claim 2 disposed in the inner space.

9. A multifunctional agent for oil, **characterized by** comprising a multifunctional agent as recited in claim 2 for use in maintaining the quality of the oil.

10. A multifunctional agent for oil according to claim 9, wherein the oil is an insulating oil.

11. A method of using a multifunctional agent, **characterized in that** the method comprises using a multifunctional agent as recited in claim 2 which is placed at least in the insulating oil or a site in the vicinity of the insulating oil.

12. An oil-immersed transformer comprising a transformer tank and an insulating oil charged in the transformer tank, **characterized in that** the multifunctional agent as recited in claim 2 is located at least in the insulating oil or a site in the vicinity of the insulating oil.

13. An oil-impregnated capacitor comprising a capacitor tank for accommodating the capacitor, and an insulating oil charged in the capacitor tank, **characterized in that** the multifunctional agent as recited in claim 2 is located at least in the insulating oil or a site in the vicinity of the insulating oil.

14. A gas phase-modifying agent, **characterized by** comprising a multifunctional agent as recited in claim 2 used for modifying a gas phase.

15. A tobacco smoke filter, **characterized by** comprising a multifunctional agent as recited in claim 2, to thereby modify tobacco smoke.

16. An attachment for tobacco smoke, **characterized by** having a casing at least having two openings and an inner space, and a multifunctional agent as recited in claim 2 disposed in the inner space, wherein a tobacco piece is attached to an opening, and the other opening serves as a mouth piece, and tobacco smoke which has been passed through the casing is to be modified.

17. A mask, **characterized by** having a filter as recited in claim 7 and a reactor as recited in claim 8.

18. A method for yielding a mayenite-compound-containing product containing a multifunctional agent as recited in claim 2, wherein the method comprises at least a mixing and pulverization step and a second firing step among a mixing and pulverization step of mixing and crushing raw materials by means a mixing and pulverization tool, to thereby obtain a mixture; a firing step of firing the mixture placed in or on a firing tool, to thereby obtain a mayenite compound precursor; a molding step of molding the mixture or the mayenite compound precursor placed in a mold, to thereby form a compact; and a second firing step of firing the mixture, the compact, or the mayenite compound precursor placed in or on a firing tool, in a reducing atmosphere or under inert gas;
wherein at least one of the mixing and pulverization tool, molding tool, and firing tool is made of the mayenite compound.

19. A method for producing a mayenite-compound-containing product according to claim 18, **characterized in that**, in the second firing step, at least one of a dehydrating agent and a deoxygenating agent is placed in at least one of a furnace and a gas feed path to the furnace, to thereby conduct firing in at least one of a dry atmosphere and a deoxygenated atmosphere.
